# EUROPEAN PATENT APPLICATION

(11) **EP 3 171 158 A1**
(43) Date of publication of application: **24.05.2017**
(21) Application number: 15822539.1
(22) Date of filing: 08.05.2015
(51) Int. Cl.: G01N 21/27, C12M 1/34, G06F 17/30

(54) **CELL OBSERVATION DATA PROCESSING SYSTEM, CELL OBSERVATION DATA PROCESSING METHOD, AND CELL OBSERVATION DATA PROCESSING PROGRAM**

(30) Priority: 16.07.2014 JP 2014145878
(71) Applicant: Olympus Corporation, Hachioji-shi Tokyo 192-8507 (JP)
(72) Inventor: IGA, Yasunobu, Tokyo 192-8507 (JP); TAKIMOTO, Shinichi, Tokyo 151-0072 (JP); TANIKAWA, Yohei, Tokyo 151-0072 (JP); AKAHORI, Yoshinobu, Tokyo 151-0073 (JP)
(74) Representative: Gunzelmann, Rainer
(86) International application number: PCT/JP2015/063325
(87) International publication number: WO 2016/009708

(57) **Abstract**

A cell observation information processing system includes: a tag acquiring section 11 that acquires, as a search tag for searching for observation information acquired via an observation information acquiring section 20 in response to an acquisition order at one time point, tags containing a cell name, first culture tool identifying information for distinguishing a culture tool for culturing cells under observation from another culture tool, and second culture tool identifying information for identifying a passage-source culture tool; a storing section 12 that stores in an archive section 13 each piece of the observation information acquired at each time point upon assigning thereto the search tag; a search criterion acquiring section 14 that acquires, as a search criterion, tags containing a cell name and a piece of first culture tool identifying information; and a retrieving section 15 that searches through the archive section using the acquired search criterion and retrieves search-tagged observation information on cells having undergone passage of culture from a single cell line over a plurality of generations.

## Description

### TECHNICAL FIELD

The present invention relates to a cell observation information processing system, a cell observation information processing method, and a cell observation information processing program, each of which is to be used for condition management of cells to be used in biological research by use of observation information (image and cell activity data) on the cells acquired with an observation information acquiring apparatus, such as a microscope.

### BACKGROUND ART

In fields where experiment and/or research is conducted by use of cells, users continue culturing cells on a daily basis and control, through observation of images of the cells under microscopes or other tools, the condition of the cells used in the experiment and/or research.

In general, cells used in experiment and/or research are separated from a living body, planted in a culture medium in a predetermined culture container, and cultured and proliferated. The cells to be cultured are called a parent cell line. When cells in a parent cell line are proliferated to a predetermined confluency or number, a part of the cells in the culture container of the parent cell line is transplanted into a culture medium in another culture container and cultured and proliferated. The process is called subculture (passage of culture). Cells for experiment and/or research separated from the parent cell line are called a subculture.

In experiment and/or research using cells, a user repeats passage of culture while proliferating the cells in the culture container containing the parent cell line and uses, for experiment and/or research, cells of a subculture separated from the subcultured parent cell line.

To increase accurateness of experiment and/or research, it is desired that cells used on individual occasions of the experiment and/or research are kept in good culture conditions.

Cell culture is classified into two types, culture for maintaining cells (hereinafter referred to as "maintenance culture") and culture for increasing the number of cells (hereinafter referred to as "expansion culture").

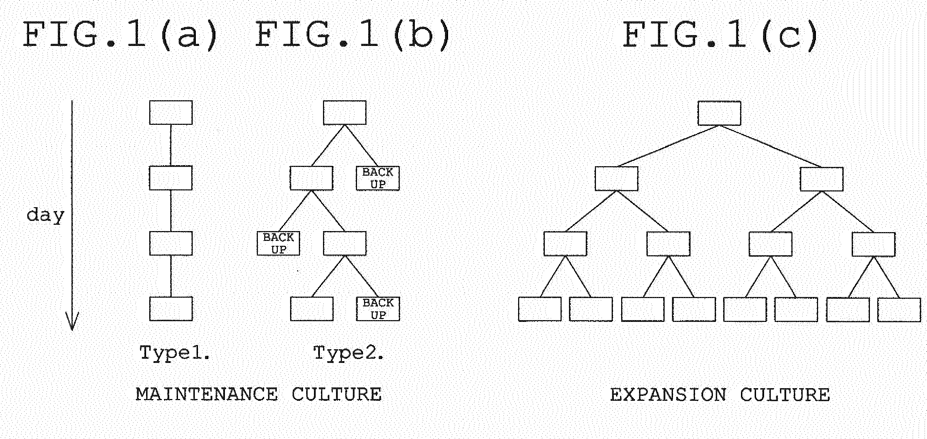

The maintenance culture is further classified into two types, maintenance culture of the type shown in FIG. 1(a) (hereinafter referred to as "type-1 maintenance culture") and maintenance culture of the type shown in FIG. 1(b) (hereinafter referred to as "type-2 maintenance culture").

For the type-1 maintenance culture, in a passage operation, a part of the cells cultured in a passage-source culture container is planted into a medium in a single new culture container. This procedure is repeated on each occasion of passage for cell proliferation.

Therefore, in the type-1 maintenance culture, source cells of a passage and destination cells of the passage are linked with each other in a one-to-one relationship, as shown in FIG. 1(a), as well as there is one line along which passage-source cells and passage-destination cells are linked together in time series over a plurality of generations.

The type-2 maintenance culture is a common type of maintenance culture. In the type-2 maintenance culture, to control loss of cells in the parent cell line culture container owing to contamination that could occur when subculture for experiment and/or research is separated from the subcultured parent cell line and to moderate fluctuation in number of cells owing to variation in cell proliferation speed, a part of the cells cultured in the passage-source culture container is planted, in a passage operation, into media in a plurality of culture containers including a culture container for a backup sample. Thereafter, in the next-time passage operation, a culture container in which cells are cultured in an optimum condition is selected among the plurality of culture containers including the culture container for a backup sample, as a passage-source culture container, and a part of the cells cultured in the selected culture container is planted into media in a plurality of culture containers including a culture container for a backup sample. The procedure described above is repeated in each passage operation for cell proliferation.

Therefore, in the type-2 maintenance culture, source cells of a passage and destination cells of the passage are linked with each other in a one-to-many relationship, as shown in FIG. 1(b). Further, although there is a line along which passage-source cells and passage-destination cells are linked with one another in time series over a plurality of generations, the cells in a culture container that is not selectedas apassage-source culture container inapassage operation is not linked with cells of later generations in time series.

For the expansion culture, in a passage operation, a part of the cells cultured in a passage-source culture container is planted into media in apluralityof culture containers, and the cells cultured in each of the culture containers are used as passage-source cells in the next-time passage operation. The procedure described above is repeated in each passage operation for cell proliferation.

Therefore, in the expansion culture, the passage-source cells and the passage-destination cells are linked with one another in a one-to-many relationship, as shown in FIG. 1(c). Further, there are a plurality of lines along each of which passage-source cells and passage-destination cells are linked with one another in time series over a plurality of generations.

In the course of culturing cells, the condition of the cells may undergo a long-term change. To grasp the long-term change in condition of the cells, it is desirable to make cell observation information at individual time points searchable by creating a database of the observation information. To narrow down the pieces of cell observation information having cell culture courses recorded therein in the database file under a desired criterion and to grasp the narrowed-down pieces of observation information in time series manner, a search tag may be assigned to observation information.

Further, to accurately grasp a long-term cell condition change that occurs in the course of the repetitive passage, for example by expressing the change in number of cells in the form of a graph, it is desired to select, among pieces of the observation information including those for backup samples, which were obtained at individual time points of observation information acquisition, only pieces of observation information on samples each selected as a passage source in a passage operation and to link the selected pieces of observation information with one another in time series.

On the other hand, there have been proposed automatic culture apparatuses having an information continuation section that associates, when a container is carried in or out of the apparatus, information before the carrying with information after the carrying, for example in the following Patent Document 1.

### PRIOR ART DOCUMENTS

### PATENT DOCUMENTS

Patent Document 1: Japanese Patent Kokai No. 2007-330145

### SUMMARY OF THE INVENTION

### PROBLEMS TO BE SOLVED BY THE INVENTION

The art described in Patent Document 1 associates information before carrying-out with information after the carrying-out in a one-to-one relationship similar to the relationship between the passage-source cells and the passage-destination cells in the type-1 maintenance culture, whereas Patent Document 1 does not disclose associating pieces of information to one another in a one-to-many relationship similar to the relationship between the passage-source cells and the passage destination cells in the type-2 maintenance culture or the expansion culture.

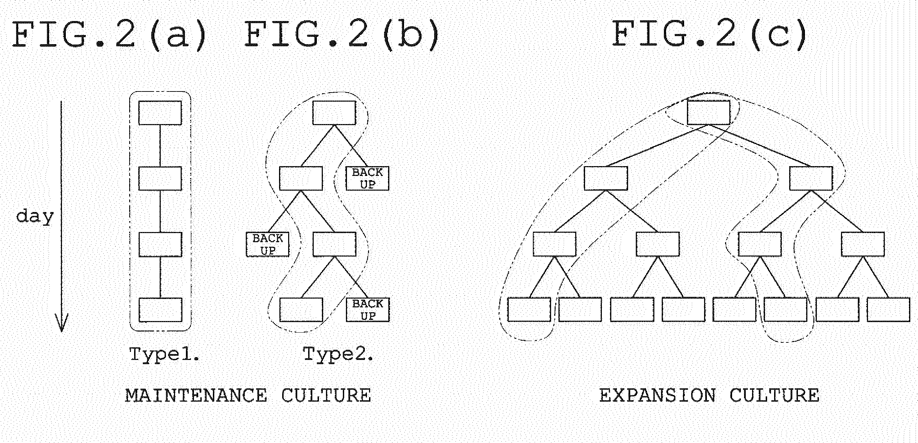

Therefore, in single-line subculture, such as the type-1 maintenance culture, applying one-to-one association of pieces of information as described in Patent Document 1 allows pieces of observation information over a plurality of generations to be linked with one another in time series, as indicated by the chain double-dashed line in FIG. 2(a), and the user can grasp the single-line observation information over a plurality of generations.

In one-to-many subculture, such as the type-2 maintenance culture and the expansion culture, however, applying the one-to-one association of pieces of information as described in Patent Document 1 does not allow pieces of observation information on cells of a particular line over a plurality of generations, such as those indicated by the chain double-dashed lines in FIGs. 2 (b) and 2(c), to be linked together in time series, and the user cannot grasp the pieces of observation information on the cells of the particular line over a plurality of generations.

That is, in the cell culture in which a part of cells cultured in a passage-source culture container is distributed to a plurality of culture containers in a passage operation and then cultured there, as in the type-2 maintenance culture and the expansion culture, there should be a plurality of culture containers in which cells having the same cell name and the same passage number are cultured. In this case, the one-to-one association of pieces of information as described in Patent Document 1 does not allow retrieval of specific-line-cell observation information formed of only pieces of observation information on cells cultured in passage-source culture containers over generations and linked with one another in time series.

The problem described above will be described below in detail.

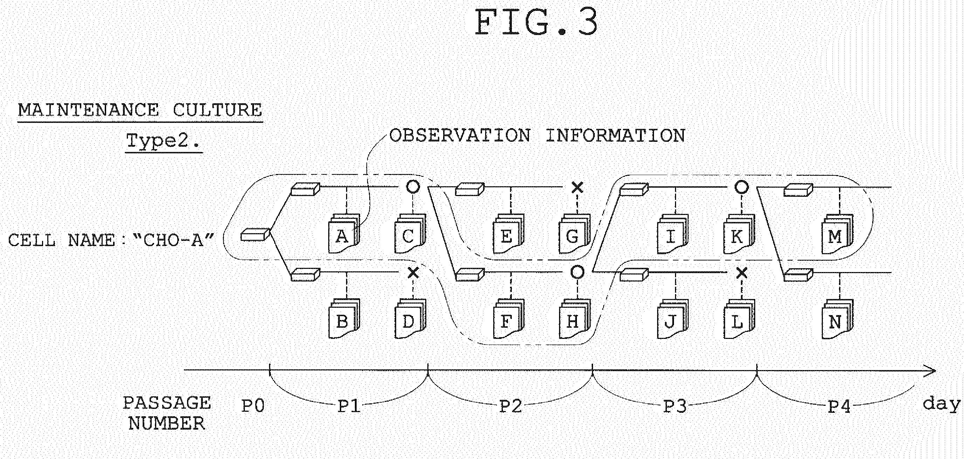

FIG. 3 is an explanatory diagram that schematically shows, in time series, passage-source- and passage-destination- culture containers and observation information at each passage number in maintenance culture of the type shown in FIG. 1(b), as an example of subculturing in which the source of a passage and the destination of the passage are associated with each other in a one-to-many relationship. It is assumed for convenience that the following description will be made with reference to the type-2 maintenance culture as passage of culture of the type in which the source of a passage and the destination of the passage are associated with one another in a one-to-many relationship.

In the example shown in FIG. 3, in the generation where the passage number is "P0", a part of the cells cultured in the passage-source culture container is distributed to two culture containers as passage-destination culture containers and then is cultured there. In the generation where the passage number is "P1", the upper one of the two culture containers is selected as a passage-source culture container, and a part of the cells cultured in the selected culture container is distributed to two culture containers at the time of passage operation and then cultured there, whereas the lower culture container is used as a culture container for a backup sample. Further, in the generation where the passage number is "P2", the lower one of the two culture containers is selected as apassage-source culture container, and a part of the cells cultured in the selected culture container is distributed to two culture containers at the time of passage operation and then cultured there, whereas the upper culture container is used as a culture container for a backup sample. Still further, in the generation where the passage number is "P3", the upper one of the two culture containers is selected as a passage-source culture container, and a part of the cells cultured in the selected culture container is distributed to two culture containers at the time of passage operation and then cultured there, whereas the lower culture container is used as a culture container for a backup sample. In FIG. 3, O represents a time point when the culture container is selected as the passage source, x represents a time point when the culture container fails to be selected as the passage source, and pieces of observation information on the cells cultured in the culture containers in the generations labeled with the passage numbers "P0", "P1", "P2", "P3", and "P4" are labeled with alphabetical letters.

In the example shown in FIG. 3, observation information on the cells having undergone passage of culture from the passage-source culture container through the generations labeled with the passage numbers "P0" to "P4" are the pieces of observation information A, C, F, H, I, K, M, and N, and as lines along each of which the passage-source cells link with passage-destination cells over a plurality of generations, there are two lines-a line along which the pieces of observation information A, C, F, H, I, K, and M link with one another and a line along which the pieces of observation information A, C, F, H, I, K, and N link with one another.

In FIG. 3, for example, when one-to-one association of pieces of information as described in Patent Document 1 is used for the purpose of retrieving only the pieces of observation information A, C, F, H, I, K, and M encircled with the chain double-dotted-line frame from a database file, the following problem occurs.

For example, in the example in FIG. 3, when passage is made from the cells in the generation expressed by the passage number "P0" to the cells in the generation expressed by the passage number "P1", a part of the cells cultured in the single culture container in the generation expressed by the passage number "P0" is distributed to two culture containers and then cultured there, as described above. At the time of passage from the cells in the generation expressed by the passage number "P0" to the cells in the generation expressed by the passage number "P1", however, it is not yet decided cells cultured in which of the two culture containers in the generation expressed by the passage number "P1" should serve as the next passage-source cells.

Therefore, according to the practice of associating pieces of information in a one-to-one relationship as described in Patent Document 1, a user is obliged to associate cells in the generation expressed by the passage number "P0" solely with cells cultured in one of the two cell containers having the passage number "P1" before it has been decided which of the cells in the two containers should serve as the next passage-source cells. Thus, the cells in the generation expressed by the passage number "P0" are prone to be associated with the cells that do not serve as the next passage source without being associated with the cells that serve as the next passage source.

If the cells in the generation expressed by the passage number "P0" are associated with the cells for which the pieces of observation information B and D would be acquired and which would fail to serve as the next passage source afterwards, they would not be linked with cells of passage number P2 and later generations. The problem possibly occurs in passage in each generation.

As described above, according to the practice of associating pieces of information in a one-to-one relationship described in Patent Document 1, it is very difficult to compile information on passage-source cells and information on cells cultured in a culture container that serves as the next passage source to be associated with each other into a database. As a result, for example, cell observation information on a particular line formed of only the pieces of observation information A, C, F, H, I, K, and M linked with one another cannot be retrieved from the database file.

Some aspects of the present invention are proposed to solve the above-mentioned conventional problem, and their object is to provide a cell observation information processing system, a cell observation information processing method, and a cell observation information processing program that allow pieces of observation information on cells having undergone passage of culture to be associated with one another over generations even for subculturing of one-to-many passage type.

Alternatively, according to some aspects of the present invention, their object is to provide a cell observation information processing system, a cell observation information processing method, and a cell observation information processing program that allow pieces of observation information on cells of a particular line having undergone passage of culture to be grasped over generations even for subculturing of one-to-many passage type.

### MEASURES TO SOLVE THE PROBLEMS

To achieve the objects described above, a cell observation information processing system according to one aspect of the present invention includes: a tag acquiring section that acquires, as a search tag for searching for a piece of observation information that has been acquired via an observation information acquiring section in response to an acquisition order at one time point to include a cell image and activity data indicating cell activity, tags containing cell identifying information including a cell name, first culture tool identifying information for distinguishing a first culture tool that is a culture tool for culturing cells under observation from another culture tool for culturing cells having the same passage number as a passage number of the cells cultured in the first culture tool, and second culture tool identifying information for identifying a second culture tool that is a culture tool for culturing cells that serve as a passage source of the cells cultured in the first culture tool; a storing section that assigns the search tag acquired by the tag acquiring section to each piece of the observation information acquired in response to an acquisition order at each time point and stores each piece of search-tagged observation information to which the search tag is assigned in an archive section; a search criterion acquiring section that acquires, as a search criterion, tags containing the cell name and the first culture tool identifying information; and a retrieving section that searches through the archive section using the search criterion acquired by the search criterion acquiring section and retrieves search-tagged observation information on cells having undergone passage of culture from a single cell line over a plurality of generations.

A cell observation information processing method according to another aspect of the present invention includes: a tag acquiring step of acquiring, as a search tag for searching for a piece of observation information that has been acquired in response to an acquisition order at one point to include a cell image and activity data indicating cell activity, tags containing cell identifying information including a cell name, first culture tool identifying information for distinguishing a first culture tool that is a culture tool for culturing cells under observation from another culture tool for culturing cells having the same passage number as a passage number of the cells cultured in the first culture tool, and second culture tool identifying information for identifying a second culture tool that is a culture tool for culturing cells that serve as a passage source of the cells cultured in the first culture tool; a storage step of assigning the search tag acquired in the tag acquiring step to each piece of the observation information acquired in response to an acquisition order at each time point and storing each piece of search-tagged observation information to which the search tag is assigned in an archive section; a search criterion acquiring step of acquiring, as a search criterion, tags containing the cell name and the first culture tool identifying information; and a retrieval step of searching through the archive section using the search criterion acquired in the search criterion acquiring step and retrieving search-tagged observation information on cells having undergone passage of culture from a single cell line over a plurality of generations.

A cell observation information processing program according to still another aspect of the present invention causes a computer to function as: tag acquiring means for acquiring, as a search tag for searching for a piece of observation information that has been acquired in response to an acquisition order at one time point to include a cell image and activity data indicating cell activity, tags containing cell identifying information including a cell name, first culture tool identifying information for distinguishing a first culture tool that is a culture tool for culturing cells under observation from another culture tool for culturing cells having the same passage number as a passage number of the cells cultured in the first culture tool, and second culture tool identifying information for identifying a second culture tool that is a culture tool for culturing cells that serve as a passage source of the cells cultured in the first culture tool; storing means for assigning the search tag acquired by the tag acquiring means to each piece of the observation information acquired in response to an acquisition order at each time point and storing each piece of search-tagged observation information to which the search tag is assigned in an archive section; search criterion acquiring means for acquiring, as a search criterion, tags containing the cell name of the first culture tool identifying information; and retrieving means for searching through the archive section using the search criterion acquired by the search criterion acquiring means and retrieving search-tagged observation information on cells having undergone passage of culture from a single cell line over a plurality of generations.

### BRIEF DESCRIPTION OF THE DRAWINGS

FIGs. 1 are explanatory diagrams that schematically shows the types of cell culture; FIG. 1 (a) shows one type of maintenance culture, FIG. 1(b) shows another type of maintenance culture, and FIG. 1(c) shows an expansion culture.
FIGs. 2 are explanatory diagrams that show examples of lines which correspond to the types of cell culture shown in FIGs. 1 and along which pieces of observation information over a plurality of generations are linked with one another in time series.
FIG. 3 is an explanatory diagram that diagrammatically shows passage-source and passage-destination culture containers and observation information at each passage number in time series in maintenance culture of the type shown in FIG. 1(b), as an example of subculturing in which the source of a passage and the destination of the passage are associated with each other in a one-to-many relationship.
FIG. 4 is a block diagram that schematically shows a basic configuration of a cell observation information processing system according to the present invention.
FIG. 5 is an explanatory diagram that shows the configuration of the cell observation information processing system according to a first embodiment of the present invention.
FIG. 6 is an explanatory diagram that shows an example of the configuration of a screen displayed in a presenting section provided in a cell observation information processing apparatus shown in FIG. 5.
FIG. 7 is an explanatory diagram that schematically shows an example of key items in a search tag designating and/or inputting field that allows a user to designate and/or input a search tag acquired by a tag acquiring section in the cell observation information processing system in FIG. 5.
FIG. 8 is an explanatory diagram that shows an example of the data structure of search-tagged observation information stored by a storing section in an archive section in the cell observation information processing system in FIG. 5.
FIG. 9 is an explanatory diagram that shows an example of the configuration of a search criterion designating and/or inputting screen that allows a user to designate and/or input a search criterion acquired by a search criterion acquiring section in the cell observation information processing system shown in FIG. 5.
FIG. 10 is a block diagram that schematically shows an example of the processing procedure at the stage of storing observation information on cells using the cell observation information processing system in FIG. 5.
FIG. 11 is a flowchart that schematically shows an example of the processing procedure at the stage of storing observation information on cells using the cell observation information processing system in FIG. 5.
FIG. 12 is a flowchart that schematically shows an example of the procedure for acquiring essential tags in the search tag acquisition process shown in FIG. 11.
FIG. 13 is a flowchart that shows an example of the processing procedure as the detail of the procedure for acquiring essential tags shown in FIG. 12.
FIG. 14 is a flowchart that shows a variation of the processing procedure in FIG. 13.
FIG. 15 is a block diagram that schematically shows an example of the processing procedure at the stage of searching for and/or retrieving desired cell observation information from the archive section using the cell observation information processing system in FIG. 5.
FIG. 16 is a flowchart that schematically shows an example of the processing procedure at the stage of searching for and/or retrieving desired cell observation information from the archive section using the cell observation information processing system in FIG. 5.
FIG. 17 is a graph that shows an example of display mode in accordance with which data including observation information retrieved by use of the cell observation information processing system in FIG. 5 is displayed in a display screen.
FIG. 18 is a flowchart that shows an example of the processing procedure for the retrieval process shown in FIG. 16 of the search-tagged observation information on cells having undergone passage of culture from a single cell line over a plurality of generations.
FIG. 19 is a flowchart that shows an example of the processing procedure in which the predetermined retrieval range of observation information in FIG. 18 is specified.
FIG. 20 is a flowchart that shows another example of the processing procedure in which the predetermined retrieval range of observation information in FIG. 18 is specified.
FIG. 21 is a flowchart that shows still another example of the processing procedure in which the predetermined retrieval range of observation information in FIG. 18 is specified.

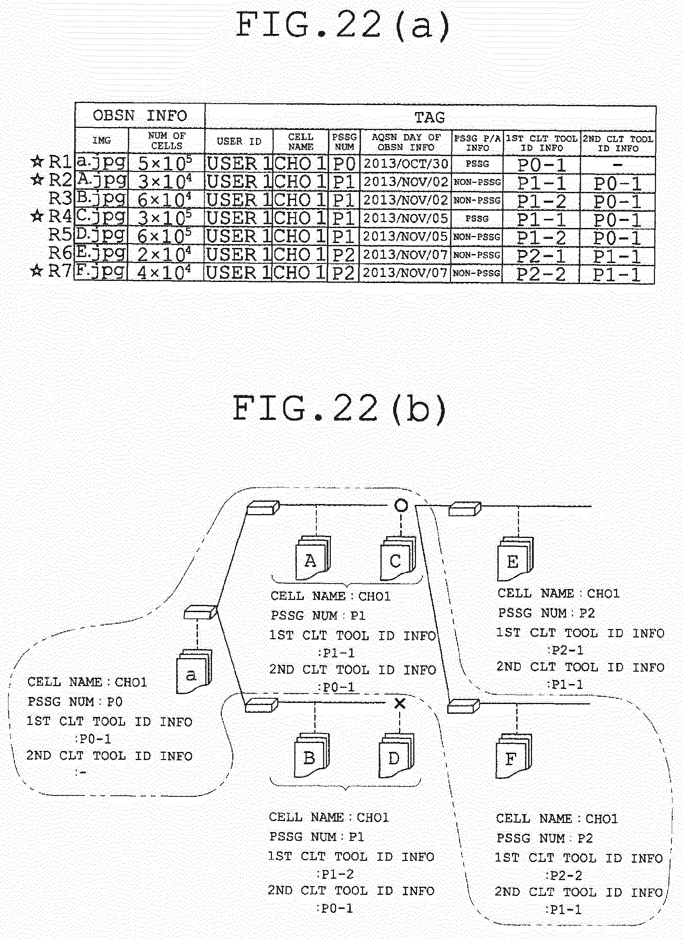

FIGs. 22 are explanatory diagrams that show an example of data and association of the data in a case where pieces of search-tagged observation information on cells over a plurality of generations are retrieved; FIG. 22(a) shows the data structure in the archive section and the data retrieved from the archive section, and FIG. 22(b) is an explanatory diagram that schematically shows passage-source and passage-destination culture containers and observation information stored in the archive section in FIG. 22 (a) in time series and associates retrieved pieces of search-tagged observation information over a plurality of generations with one another in time series.
FIG. 23 is an explanatory diagram that shows the configuration of a cell observation information processing system according to a second embodiment of the present invention.
FIG. 24 is an explanatory diagram that shows an example of the data structure of tag acquisition history information stored in the archive section in the cell observation information processing system in FIG. 23.
FIG. 25 is a flowchart that shows an example of the processing procedure as the details of the procedure for acquiring essential tags in the search tag acquisition process in the processing procedure at the stage of storing observation information on cells using the cell observation information processing system shown in FIG. 23.
FIG. 26 is a flowchart that shows a variation of the processing procedure in FIG. 25.
FIG. 27 is a flowchart that shows an example of the processing procedure for retrieval process of the search-tagged observation information on cells having undergone passage of culture from a single cell line over a plurality of generations, at the stage of searching for and/or retrieving observation information on desired cells from the archive section using the cell observation information processing system in FIG. 23.
FIG. 28 is a flowchart that shows an example of the processing procedure in which the predetermined retrieval range of observation information in FIG. 27 is specified.
FIG. 29 is a flowchart that shows another example of the processing procedure in which the predetermined retrieval range of observation information in FIG. 27 is specified.
FIG. 30 is a flowchart that shows still another example of the processing procedure in which the predetermined retrieval range of observation information in FIG. 27 is specified.

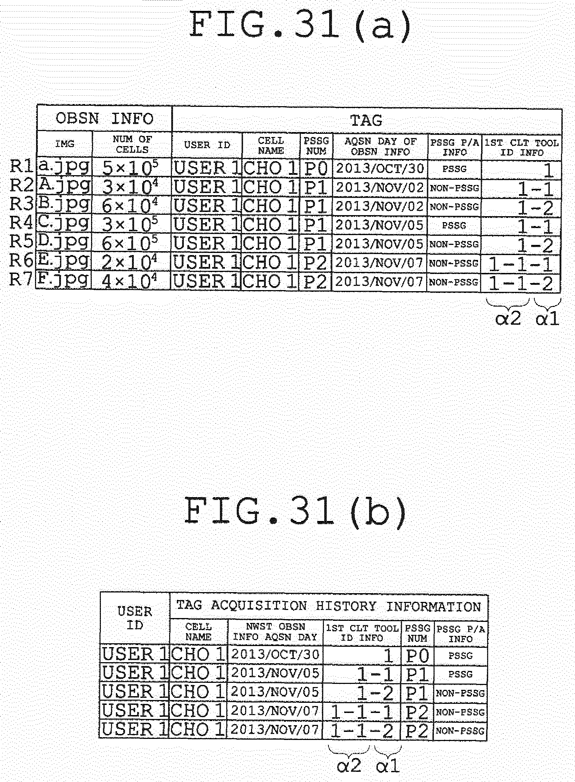

FIGs. 31 are explanatory diagrams that show examples of the data structure of first culture tool identifying information and second culture tool identifying information stored in an archive section in a cell observation information processing system according to a third embodiment of the present invention; FIG. 31(a) shows an example of the data structure of the first culture tool identifying information and the second culture tool identifying information in search-tagged observation information, and FIG. 31(b) shows an example of the data structure of the first culture tool identifying information and the second culture tool identifying information in tag acquisition history information.
FIG. 32 is a flowchart that shows an example of the processing procedure of the search-tagged observation information retrieval process on cells having undergone passage of culture from a single cell line over a plurality of generations, at the stage of searching for and/or retrieving observation information on desired cells from the archive section using the cell observation information processing system in FIGs. 31.
FIGs. 33 are explanatory diagrams that schematically show an example of a cell culture container tree diagram on container basis by using tagged observation information stored in the archive section and on the basis of a group of pieces of observation information having the same user ID, cell name, passage number, first culture tool identifying information, and second culture tool identifying information.

### MODE FOR CARRYING OUT THE INVENTION

Embodiments of the present invention will be described below. It is not intended that the embodiments described below unduly limit the contents of the present invention set forth in the claims. Further, all configurations described in the following embodiments are not necessarily essential configuration requirements of the present invention.

### (1) Basic configuration and functions and effects

A cell observation information processing system according to an embodiment of the present invention includes: a tag acquiring section that acquires, as a search tag for searching for a piece of observation information that has been acquired via an observation information acquiring section in response to an acquisition order at one time point to include a cell image and activity data indicating cell activity, tags containing cell identifying information including a cell name, first culture tool identifying information for distinguishing a first culture tool that is a culture tool for culturing cells under observation from another culture tool for culturing cells having the same passage number as a passage number of the cells cultured in the first culture tool, and second culture tool identifying information for identifying a second culture tool that is a culture tool for culturing cells that serve as a passage source of the cells cultured in the first culture tool; a storing section that assigns the search tag acquired by the tag acquiring section to each piece of the observation information acquired in response to the acquisition order at each time point and stores, in an archive section, search-tagged observation information to which the search tag is assigned; a search criterion acquiring section that acquires tags containing the cell name and the first culture tool identifying information as a search criterion; and a retrieving section that searches through the archive section using the search criterion acquired by the search criterion acquiring section and retrieves search-tagged observation information on cells having undergone passage of culture from a single cell line over a plurality of generations.

The culture tool may be any tool that allows cell culture. Examples of the culture tool may include culture containers such as a flask, a dish and a well plate, and a slide glass plate.

The activity data indicating cell activity may include the number of cells, cell confluency, morphology, viability, and other factors of the cells.

The observation information is information indicating a result of observation of cells. Examples of the observation information may include an observation name as well as the cell image and the activity data.

The cell identifying information is identification information for identifying cells under observation. In more detail, examples of the cell identifying information may include the passage number of the cells, the kind of the cells, and cell level information for identifying whether the cells are of the parent cell line to be cultured or a new cell line separated from and cut out of the parent cell line as well as the cell name, the first culture tool identifying information and the second culture tool identifying information.

The cell name is an arbitrary name attached to cells used by a user to conduct experiment or research, and the cell type is a specific category into which the cells are classified.

In the cell observation information processing system according to the embodiment of the present invention, the tag acquiring section acquires tags containing a cell name, first culture tool identifying information, and second culture tool identifying information a as search tag, and the storing section stores, in the archive section, search-tagged observation information to which the search tag is assigned, whereby the first culture tool identifying information provided in the search tag in the search-tagged observation information stored in the archive section allows a plurality of culture tools having the same cell name and the same passage number to be distinguished from one another, and the second culture tool identifying information allows observation information on passage-destination cells and observation information on passage-source cells to be associated with each other over a plurality of generations. The search criterion acquiring section then acquires tags containing the cell name and the first culture tool identifying information as the search criterion, and the retrieving section searches through the archive section using the search criterion acquired by the search criterion acquiring section and retrieves search-tagged observation information on cells having undergone passage of culture from a single cell line over a plurality of generations, whereby the user can grasp observation information on a particular line of cells over generations even in subculturing of the one-to-many passage type.

The embodiments of the present invention then provide the following effects in accordance with the specific configurations of the retrieving section, the search criterion acquiring section and other sections:

### (1-1) Functions and effects provided by configuration in which retrieving section repeats process of re-searching through archive section using search criterion in which second culture tool identifying information included in retrieved search-tagged observation information replaces and serves as first culture tool identifying information in search criterion and retrieving matched search-tagged observation information

In the cell observation information processing system, according to the embodiment of the present invention, the retrieving section is preferably configured to repeat, on the basis of a predetermined retrieval range of observation information, the process of searching through the archive section using the search criterion acquired by the search criterion acquiring section, retrieving matched search-tagged observation information, then re-searching through the archive section using a search criterion in which the second culture tool identifying information included in the retrieved search-tagged observation information replaces and serves as the first culture tool identifying information in the search criterion, and retrieving matched search-tagged observation information.

As a result, retrieval of search-tagged observation information on cells having undergone passage of culture from a single cell line over a plurality of generations can be achieved by the retrieving section.

### (1-1-1) Functions and effects provided by configuration in which predetermined retrieval range of observation information is configured so that observation information is retrieved until search-tagged observationinformation having no second culture tool identifying information recorded therein is retrieved

In the cell observation information processing system according to the embodiment of the present invention, the predetermined retrieval range of the observation information is preferably configured so that observation information is retrieved until search-tagged observation information having no second culture tool identifying information recorded therein is retrieved.

As a result, retrieval of search-tagged observation information on cells over a plurality of generations can be achieved up to the oldest passage source generation traceable in the database.

### (1-1-2) Functions and effects provided by configuration in which predetermined retrieval range of observation information is designated by user's operation

In the cell observation information processing system according to the embodiment of the present invention, the search tag preferably further contains date/time information on observation information acquisition time point, and the predetermined retrieval range of observation information is configured to be defined by a range, designated by user's operation, of date/time information on observation information acquisition time point.

Alternatively, in the cell observation information processing system according to the embodiment of the present invention, the search tag contains a passage number of the cells cultured in the first culture tool, and the predetermined retrieval range of observation information is configured to be defined by a range of passage number designated by user's operation.

As a result, retrieval of search-tagged observation information on cells over a plurality of generations can be achieved over a range necessary for the user.

### (1-2) Functions and effects provided by configuration in which first culture tool identifying information is formed of passage number of cells cultured in first culture tool and identification information specific to first culture tool

In the cell observation information processing system according to the embodiment of the present invention, the first culture tool identifying information is preferably formed of the passage number of the cells cultured in the first culture tool and identification information specific to the first culture tool.

This configuration allows, upon the second culture tool identifying information being configured to be formed of the passage number of the cells cultured in the second culture tool and identification information specific to the second culture tool as described later, the retrieving section to retrieve search-tagged observation information on cells having a passage number one generation earlier only by replacing the first culture tool identifying information in the search criterion with a value of the second culture tool identifying information contained in the retrieved data without separately setting, as a part of the search criterion to be used for search through the archive section by the retrieving section, a passage number yielded by subtracting 1 from the current passage number.

### (1-3) Functions and effects provided by configuration in which second culture tool identifying information is formed of passage number of cells cultured in second culture tool and identification information specific to second culture tool

In the cell observation information processing system according to the embodiment of the present invention, the second culture tool identifying information is preferably formed of the passage number of the cells cultured in the second culture tool and identification information specific to the second culture tool.

This configuration allows the retrieving section to set, for retrieving search-tagged observation information on cells having a passage number one generation earlier, a value of the passage number involved in the second culture tool identifying information provided in the retrieved data as a part of the search criterion to be used for search through the archive section, without separately setting a passage number yielded by subtracting 1 from the current passage number. Search-tagged observation information on cells having a passage number one generation earlier can therefore be retrieved with a search criterion in which the first culture tool identifying information in the search criterion is replaced with a value of the identification information specific to the second culture tool involved in the second culture tool identifying information included in the retrieved data.

Further, as described above, the configuration in which the first culture tool identifying information is formed of the passage number of the cells cultured in the first culture tool and identification information specific to the first culture tool allows retrieval of search-tagged observation information on cells having a passage number one generation earlier only by replacing the first culture tool identifying information in the search criterion with a value of the second culture tool information included in the retrieved data.

### (1-4) Configuration that allows tag acquiring section to acquire first culture tool identifying information and second culture tool identifying information and functions and effects of the configuration

### (1-4-1) Functions and effects provided by configuration in which tag acquiring section searches through archive section by use of cell name designated by user, outputs, to presenting section in form of list as possible first culture tool identifying information, first culture tool identifying information included in search-tagged observation information having largest passage number of cells cultured in first culture tool among pieces of matched search-tagged observation information, acquires, as part of search tag, first culture tool identifying information selected by user, and automatically acquires, as part of search tag, second culture tool identifying information included in search-tagged observation information having first culture tool identifying information

In the cell observation information processing system according to the embodiment of the present invention, the search tag preferably further contains a passage number of the cells cultured in the first culture tool. The tag acquiring section searches through the archive section by use of a cell name designated by user's operation, retrieves search-tagged observation information having a largest passage number of the cells cultured in the first culture tool among pieces of matched search-tagged observation information, and outputs, to the presenting section, the first culture tool identifying information included in the retrieved search-tagged observation information in the form of a list as pieces of possible first culture tool identifying information to be acquired as a part of the search tag. The tag acquiring section then acquires, as a part of the search tag, the first culture tool identifying information selected by user's operation from the possible first culture tool identifying information output to the presenting section in the form of a list and automatically acquires, as a part of the search tag, the second culture tool identifying information included in the search-tagged observation information having the first culture tool identifying information selected by user's operation.

User's operation of typing keys to input the first culture tool identifying information and the second culture tool identifying information can be therefore omitted, and an error of key typing can also be eliminated. As a result, the efficiency of search tag acquisition can be improved, and the time taken to check the condition of the cells can be shortened.

In particular, in a case where the condition of the cells is checked with the cells being taken out of an incubator, the time taken to check the condition of the cells is shortened, whereby the amount of damage on the cells can be reduced.

### (1-4-2) Functions and effects provided by configuration in which tag acquiring section acquires, as part of search tag, first culture tool identifying information designated by user, searches through archive section using cell name and first culture tool identifying information designated by user, and automatically acquires, as part of search tag, second culture tool identifying information included in matched search-tagged observation information

In the cell observation information processing system according to the embodiment of the present invention, the tag acquiring section is preferably configured to acquire, as a part of the search tag, first culture tool identifying information designated by user's operation, search through the archive section using a cell name and the first culture tool identifying information designated by user's operation, and if matched search-tagged observation information is present, automatically acquire, as a part of the search tag, the second culture tool identifying information included in the search-tagged observation information.

As a result, user's operation of typing keys to input the second culture tool identifying information can be omitted, and an error of key typing can also be eliminated. Further, since operation of typing keys to input the second culture tool identifying information is omitted, the efficiency of search tag acquisition is improved accordingly, whereby the time taken to check the condition of the cells can be shortened.

### (1-4-3) Functions and effects provided by configuration in which, if new culture tool identifying information is designated by user as first culture tool identifying information, tag acquiring section acquires, as part of search tag, first culture tool identifying information designated by user, searches through archive section using cell name designated by user, outputs, to presenting section in form of list as possible second culture tool identifying information, first culture tool identifying information included in search-tagged observation information having latest observation information acquisition time point with passage presence/absence information indicating passage work among pieces of matched search-tagged observation information, and acquires, as part of search tag, second culture tool identifying information selected by user

In the cell observation information processing system according to the embodiment of the present invention, the search tag preferably further includes passage presence/absence information indicating whether or not operation performed on first cells is passage work and date/time information at an observation information acquisition time point. When new culture tool identifying information is designated by user's operation as first culture tool identifying information to be acquired as a part of the search tag, the tag acquiring section acquires, as a part of the search tag, the first culture tool identifying information designated by user's operation, searches through the archive section using a cell name designated by user's operation, retrieves, among pieces of matched search-tagged observation information, search-tagged observation information that has passage presence/absence information indicating passage work as well as has a latest observation information acquisition time point, and outputs, to the presenting section in the form of a list, first culture tool identifying information included in the retrieved search-tagged observation information as possible second culture tool identifying information to be acquired as a part of the search tag. The tag acquiring section then acquires, amongpieces of the possible second culture tool identifying information output to the presenting section in the form of a list, second culture tool identifying information selected by user's operation as a part of the search tag.

Also in this case, user's operation of typing keys to input the second culture tool identifying information can be omitted, and an error of key typing can also be eliminated. Further, since operation of typing keys to input the second culture tool identifying information is omitted, the efficiency of search tag acquisition is improved accordingly, whereby the time taken to check the condition of the cells can be shortened.

### (1-4-4) Functions and effects provided by configuration in which tag acquiring section acquires, as part of search tag, first culture tool identifying information designated by user, searches through archive section using cell name and first culture tool identifying information designated by user, further searches, if no matched search-tagged observation information is present, through archive section using cell name designated by user, outputs, to presenting section in form of list as possible second culture tool identifying information, first culture tool identifying information included in search-tagged observation information having latest observation information acquisition time point with passage presence/absence information indicating passage work among pieces of matched search-tagged observation information, and acquires, as part of search tag, second culture tool identifying information selected by user

In the cell observation information processing system according to the embodiment of the present invention, the search tag preferably further contains passage presence/absence information indicating whether or not operation performed on the cells cultured in the first culture tool is passage work and the date/time information at an observation information acquisition time point. The tag acquiring section is configured to acquire, as a part of the search tag, the first culture tool identifying information designated by user's operation, search through the archive section using a cell name and the first culture tool identifying information designated by user's operation, further search, if no matched search-tagged observation information is present, through the archive section using the cell name designated by user's operation, retrieve, among pieces of matched search-tagged observation information, search-tagged observation information that has passage presence/absence information indicating passage work as well as has a latest observation information acquisition time point, outputs, to the presenting section in the form of a list, first culture tool identifying information included in the retrieved search-tagged observation information as possible second culture tool identifying information to be acquired as a part of the search tag, and acquire, among pieces of the possible second culture tool identifying information output to the presenting section in the form of a list, second culture tool identifying information selected by user's operation as a part of the search tag.

Also in this case, user's operation of typing keys to input the second culture tool identifying information can be omitted, and an error of key typing can also be eliminated. Further, since operation of typing keys to input the second culture tool identifying information is omitted, the efficiency of search tag acquisition is improved accordingly, whereby the period required to check the condition of cells can be shortened.

### (1-5) Functions and effects provided by configuration in which first culture tool identifying information and second culture tool identifying information are integrated together

In the cell observation information processing system according to the embodiment of the present invention, it is preferably configured so that the first culture tool identifying information involves the second culture tool identifying information, and so that, after searching through the archive section using the search criterion acquired by the search criterion acquiring section and retrieving matched search-tagged observation information, the retrieving section further searches through the archive section sequentially using search criteria in which the second culture tool identifying information of respective generations involved in the first culture tool identifying information in the search criterion replaces and serves as first culture tool identifying information in search criterion, to retrieve matched search-tagged observation information.

In the cell observation information processing system according to the embodiment of the present invention, in which the first culture tool identifying information and the second culture tool identifying information are integrated together, data in a certain piece of first culture tool identifying information allow identification of the first culture tool identifying information and the second culture tool identifying information on all-generation cells that link with one another retrospectively from the generation of the passage number concerned. The retrieving section therefore does not need, whenever carrying out the process of searching through the archive section using a search criterion and retrieving matched search-tagged observation information, to carry out the process of acquiring the second culture tool identifying information included in the retrieved search-tagged observation information but can continuously search through the archive section using search criteria in each of which second culture tool identifying information in each generation identified in the data of the first culture tool identifying information contained in the search criterion acquired by the search criterion acquiring section replaces the first culture tool identifying information in the search criterion, to retrieve matched search-tagged observation information. Further, the search criterion used by the retrieving section to retrieve, back to earlier generations, search-tagged observation information from the archive section need not include information on the passage number. As a result, the efficiency of the process of retrieving observation information on cells having undergone passage of culture from a single cell line over a plurality of generations is significantly improved.

### (2) Functions and effects provided by configuration in which storing section stores first culture tool identifying information and second culture tool identifying information as tag acquisition history information in file separate from file for storing search-tagged observation information

In the cell observation information processing system according to the embodiment of the present invention, the storing section is preferably configured to store, as tag acquisition history information, tags containing cell identifying information including a cell name, first culture tool identifying information, and second culture tool identifying information acquired by the tag acquiring section as the search tag, in a file separate from the file for storing search-tagged observation information in the archive section.

The search-tagged observation information contains large-byte data, such as a cell image. Therefore, the processing efficiency of search and/or retrieval of the search-tagged observation information carried out in the course of the search tag acquisition process by the tag acquiring section and the search-tagged observation information retrieval process by the retrieving section decreases because of the large-byte data.

In the cell observation information processing system according to the embodiment of the present invention, the storing section stores, as tag acquisition history information, tags containing cell identifying information including a cell name, first culture tool identifying information, and second culture tool identifying information acquired by the tag acquiring section as the search tag, in a file separate from the file for storing search-tagged observation information. Therefore, in the search tag acquisition process by the tag acquiring section and the search-tagged observation information retrieval process by the retrieving section, a target to be searched for and/or retrieved can be a file that stores tag acquisition history information having no large-byte data such as a cell image, whereby the efficiency of search and/or retrieval process can be improved.

### (2-1) Functions and effects provided by configuration in which retrieving section repeats, on basis of predetermined retrieval range of tag acquisition history information, process of newly creating search criterion in which second culture tool identifying information included in retrieved tag acquisition history information replaces and serve as first culture tool identifying information in search criterion, re-searching through archive section using created search criterion, and retrieving matched tag acquisition history information, then sequentially searches through archive section using all search criteria used to retrieve tag acquisition history information on basis of predetermined retrieval range of tag acquisition history information, and retrieves matched search-tagged observation information

In the cell observation information processing system according to the embodiment of the present invention, the retrieving section is preferably configured to repeat, on the basis of a predetermined retrieval range of tag acquisition history information, the process of searching through the archive section using the search criterion acquired by the search criterion acquiring section, retrieving matched tag acquisition history information, then further newly creating a search criterion in which the first culture tool identifying information in the search criterion is replaced with the second culture tool identifying information included in the retrieved tag acquisition history information, re-searching for the archive section using the created search criterion, and retrieving matched tag acquisition history information, then sequentially search through the archive section using all the search criteria used to retrieve the tag acquisition history information on the basis of the predetermined acquisition history information retrieval range, and retrieve matched search-tagged observation information.

As a result, not only retrieval of search-tagged observation information on cells having undergone passage of culture from a single cell line over a plurality of generations by the retrieving section can be achieved, but also efficiency of the search and/or retrieval process in the processing course of the search-tagged observation information retrieving process carried out by the retrieving section is improved.

### (2-1-1) Functions and effects provided by configuration in which predetermined retrieval range of tag acquisition history information is configured so that tag acquisition history information is retrieved until tag acquisition history information having no second culture tool identifying information recorded therein is retrieved

In the cell observation information processing system according to the embodiment of the present invention, the predetermined acquisition history information retrieval range is preferably configured so that tag acquisition history information is retrieved until tag acquisition history information having no second culture tool identifying information recorded therein is retrieved.

As a result, search-tagged observation information on cells over a plurality of generations can be retrieved back to the oldest passage source generation traceable in the database.

### (2-1-2) Functions and effects provided by configuration in which predetermined acquisition history information retrieval range is designated by user's operation

In the cell observation information processing system according to the embodiment of the present invention, the search tag preferably further contains date/time information at an observation information acquisition time point, and the predetermined acquisition history information retrieval range is configured to be the date/time information range determined by an observation information acquisition time point designated by user's operation.

Alternatively, in the cell observation information processing system according to the embodiment of the present invention, the search tag contains the passage number of the cells cultured in the first culture tool, and the predetermined acquisition history information retrieval range is configured to be a passage number range designated by user's operation.

As a result, search-tagged observation information on cells over a plurality of generations can be retrieved over a range necessary for the user.

### (2-2) Configuration that allows tag acquiring section to acquire first culture tool identifying information and second culture tool identifying information included in tag acquisition history information and functions and effect of the configuration (2-2-1) Functions and effects provided by configuration in which tag acquiring section searches through archive section using cell name designated by user, outputs, to presenting section in form of list as possible first culture tool identifying information, first culture tool identifying information included in tag acquisition history information having largest passage number of cells cultured in first culture tool among matched tag acquisition history information, acquires, as part of search tag, first culture tool identifying information selected by user, and automatically acquires, as part of search tag, second culture tool identifying information included in tag acquisition history information having first culture tool identifying information

In the cell observation information processing system according to the embodiment of the present invention, the search tag preferably further contains the passage number of the cells cultured in the first culture tool. The tag acquiring section searches through the archive section using a cell name designated by user's operation, retrieves tag acquisition history information having a largest passage number of cells cultured in the first culture tool among matched tag acquisition history information, and outputs, to the presenting section in the form of a list, the first culture tool identifying information included in the retrieved search-tagged observation information as pieces of possible first culture tool identifying information to be acquired as a part of the search tag. The tag acquiring section then acquires, as a part of the search tag, first culture tool identifying information selected by user's operation from the pieces of possible first culture tool identifying information output to the presenting section and automatically acquires, as a part of the search tag, second culture tool identifying information included in the tag acquisition history information having the first culture tool identifying information selected by user's operation.

User's operation of typing keys to input the first culture tool identifying information and the second culture tool identifying information can be therefore omitted, and an error of key typing can also be eliminated. As a result, the efficiency of search tag acquisition can be improved, and the time taken to check the condition of the cells can be shortened. In addition, the efficiency of the search and/or retrieval process in the processing course of the search tag acquisition process carried out by the tag acquiring section is improved.

### (2-2-2) Functions and effects provided by configuration in which tag acquiring section acquires, as part of search tag, first culture tool identifying information designated by user, searches through archive section using cell name and first culture tool identifying information designated by user, and automatically acquires, as part of search tag, second culture tool identifying information included in matched tag acquisition history information

In the cell observation information processing system according to the embodiment of the present invention, the tag acquiring section is preferably configured to acquire, as a part of the search tag, first culture tool identifying information designated by user's operation, search through the archive section using a cell name and the first culture tool identifying information designated by user's operation, and, if matched tag acquisition history information is present, automatically acquire, as a part of the search tag, second culture tool identifying information included in the matched tag acquisition history information.

As a result, user's operation of typing keys to input the second culture tool identifying information can be omitted, and an error of key typing can also be eliminated. Further, since operation of typing keys to input the second culture tool identifying information is omitted, the efficiency of search tag acquisition is improved accordingly, whereby the time taken to check the condition of the cells can be shortened. In addition, the efficiency of the search and/or retrieval process in the processing course of the search tag acquisition process carried out by the tag acquiring section is improved.

### (2-2-3) Functions and effects provided by configuration in which, if new culture tool identifying information is designated by user as first culture tool identifying information, tag acquiring section acquires, as part of search tag, first culture tool identifying information designated by user, searches through archive section using cell name designated by user, outputs, to presenting section in form of list as possible second culture tool identifying information, first culture tool identifying information included in tag acquisition history information having passage presence/absence information indicating passage work and latest observation information acquisition time point among matched tag acquisition history information, and acquires, as part of search tag, second culture tool identifying information selected by user's operation

In the cell observation information processing system according to the embodiment of the present invention, a search tag preferably further contains passage presence/absence information indicating whether or not operation performed on first cells is passage work and date/time information at an observation information acquisition time point. If new culture tool identifying information is designated by user's operation as first culture tool identifying information to be acquired as a part of the search tag, the tag acquiring section acquires, as a part of the search tag, the first culture tool identifying information designated by user's operation, searches through the archive section using a cell name designated by user's operation, retrieves tag acquisition history information having the passage presence/absence information indicating passage work as well as the latest observation information acquisition time point in the date/time information among matched tag acquisition history information, outputs to the presenting section in the form of a list as possible second culture tool identifying information to be acquired as a part of the search tag, first culture tool identifying information included in the retrieved tag acquisition history information. The tag acquiring section then acquires, among pieces of the possible second culture tool identifying information output to the presenting section in the form of a list, second culture tool identifying information selected by user's operation as a part of the search tag.

Also in this case, user's operation of typing keys to input the second culture tool identifying information can be omitted, and an error of key typing can also be eliminated. Further, since operation of typing keys to input the second culture tool identifying information is omitted, the efficiency of search tag acquisition is improved accordingly, whereby the time taken to check the condition of the cells can be shortened. In addition, the efficiency of the search and/or retrieval process in the processing course of the search tag acquisition process carried out by the tag acquiring section is improved.

### (2-2-4) Functions and effects provided by configuration in which tag acquiring section acquires, as part of search tag, first culture tool identifying information designated by user, searches through archive section using cell name and first culture tool identifying information designated by user, further searches, if no matched tag acquisition historyinformationispresent, through archive section using cell name designated by user, outputs, to presenting section in form of list as possible second culture tool identifying information, first culture tool identifying information included in tag acquisition history information having passage presence/absence information indicating passage work and latest observation information acquisition time point, and acquires, as part of search tag, second culture tool identifying information selected by user

In the cell observation information processing system according to the embodiment of the present invention, a search tag preferably further contains passage presence/absence information indicating whether or not operation performed on the cells cultured in the first culture tool is passage work and the date/time information at an observation information acquisition time point. The tag acquiring section is configured to acquire, as a part of the search tag, the first culture tool identifying information designated by user's operation, search through the archive section using a cell name and the first culture tool identifying information designated by user's operation, further search, if no matched tag acquisition history information is present, through the archive section using the cell name designated by user' s operation, retrieve tag acquisition history information having passage presence/absence information indicating passage work and the latest observation information acquisition time point in the date/time information among pieces of the matched tag acquisition history information, output, to the presenting section, first culture tool identifying information included in the retrieved tag acquisition history information in the form of a list as possible second culture tool identifying information to be acquired as a part of the search tag, and acquire, among pieces of the second culture tool identifying information output to the presenting section in the form of a list, the second culture tool identifying information selected by user's operation as a part of the search tag.

Also in this case, user's operation of typing keys to input the second culture tool identifying information can be omitted, and an error of key typing can also be eliminated. Further, since operation of typing keys to input the second culture tool identifying information is omitted, the efficiency of search tag acquisition is improved accordingly, whereby the time taken to check the condition of cells can be shortened. In addition, the efficiency of the search and/or retrieval process in the processing course of the search tag acquisition process carried out by the tag acquiring section is improved.

### (3) Others

In the cell observation information processing system according to each of the embodiments of the present invention, the search tag preferably contains user identifying information (user oneself or acting operator).

The user identifying information is identification information for identifying the user who performs cell observation, in more detail, identification information for identifying the very person concerned who inputs results of the cell observation or an acting person who observes cells and inputs results of the cell observation in place of the very person concerned.

In this case, even in a situation in which a plurality of users manage cells to which the same cell name is assigned, in selecting first culture tool identifying information from the list thereof displayed by the presenting section in the search tag acquisition process carried out by the tag acquiring section, only the first culture tool identifying information assigned to observation information under the person's own control can be output in the form of a list, and first culture tool identifying information assigned to observation information under other persons' control and hence required not to be referred to can be excluded from the information to be output to the presenting section. As a result, misselection by the user of first culture tool identifying information to be assigned as a part of the search tag to observation information can be avoided, and the efficiency of the selection is improved. As a result, the efficiency of the search tag acquisition can be further improved, and the time taken to check the cell condition can be further shortened.

Further, in the processing course of the search-tagged observation information retrieving process carried out by the retrieving section and other processes, observation information on cells under other persons' control and unnecessary for search and/or retrieval can be excluded. As a result, even in the situation in which a plurality of users manage cells to which the same cell name is assigned, a person can accurately grasp observation information on specific-line cells over a plurality of generations only under the person's own control.

Embodiments of the present invention will be described below with reference to the drawings.

### First embodiment

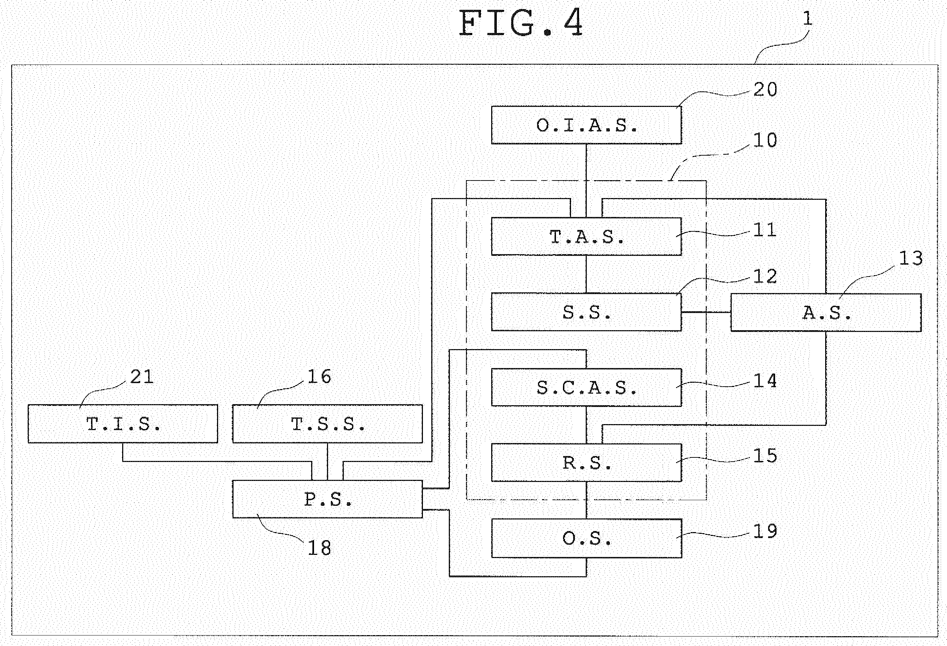

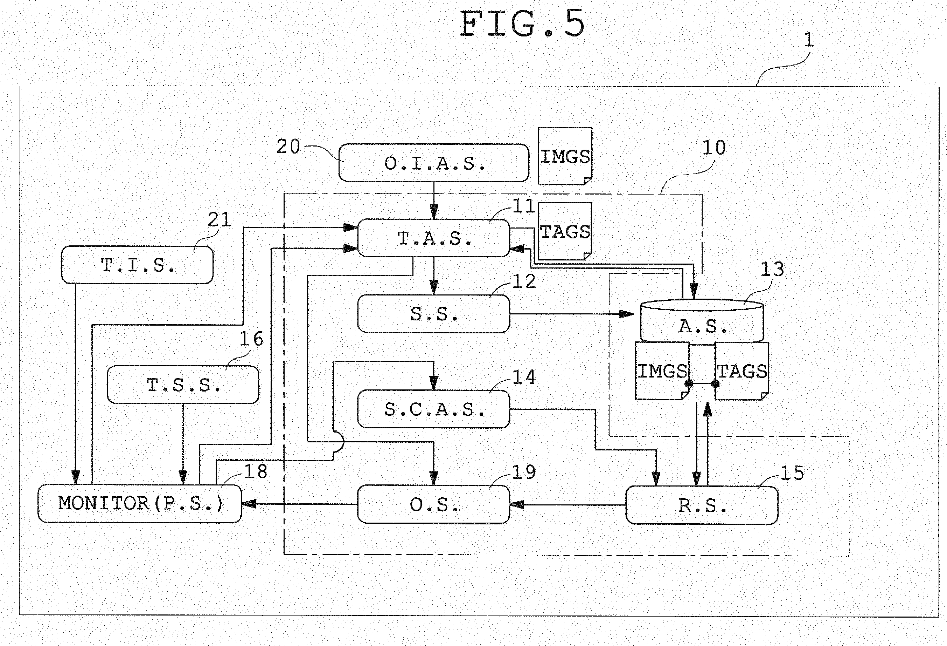

FIG. 4 is a block diagram that schematically shows a basic configuration of a cell observation information processing system according to the present invention. FIG. 5 is an explanatory diagram that shows the configuration of the cell observation information processing system according to a first embodiment of the present invention.

A cell observation information processing system 10 according to the first embodiment includes a tag acquiring section 11, a storing section 12, a search criterion acquiring section 14, and a retrieving section 15, as shown in FIGs. 4 and 5. In FIGs. 4 and 5, the reference numeral 1 denotes a cell observation information processing installation, the reference numeral 13 denotes an archive section, the reference numeral 16 denotes a tag selecting section, the reference numeral 18 denotes a monitor as a presenting section, the reference numeral 19 denotes an outputting section, the reference numeral 20 denotes an observation information acquiring section, and the reference numeral 21 denotes a tag inputting section. In FIG. 5, the cell observation information processing system 10 has a standalone configuration in which the tag acquiring section 11, the storing section 12, the search criterion acquiring section 14, and the retrieving section 15 along with the archive section 13, the tag selecting section 16, the tag inputting section 21, the presenting section 18, the outputting section 19, and the observation information acquiring section 20 are accommodated in the single installation 1. The cell observation information processing installation 1 is formed, for example, of a microscope apparatus including an imaging device and a computer provided with a database.

The tag acquiring section 11 is configured to acquire, as a search tag for searching for observation information that has been acquired via the observation information acquiring section 20 in response to an acquisition order at one time point to include a cell image and activity data indicating cell activity, tags containing cell identifying information including a cell name, first culture tool identifying information for distinguishing a first culture tool that is a culture tool for culturing cells under observation from another culture tool for culturing cells having the same passage number as that of the cells under observation, and second culture tool identifying information for identifying a second culture tool that is a culture tool for culturing passage-source cells from which the cells cultured in the first culture tool are created. The observation information, the cell name, the culture tool, and the cell identifying information have been described above.

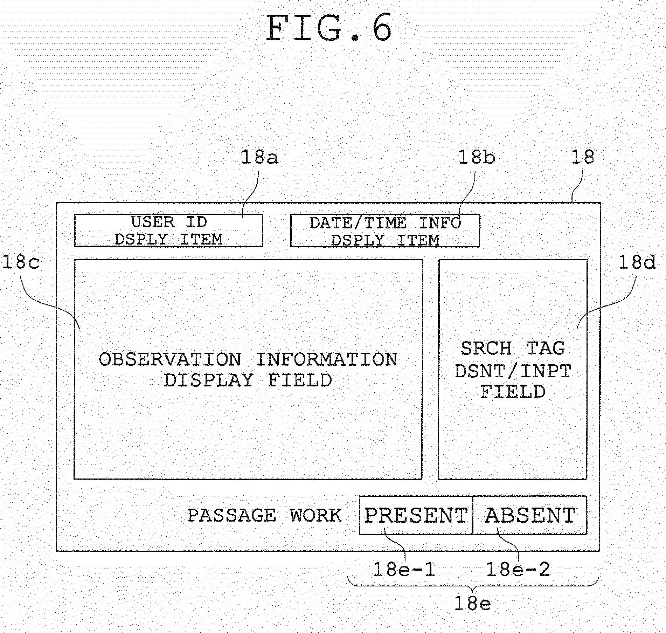

To be specific, the tag acquiring section 11 is configured to acquire, as a search tag, tags containing a user ID as user identifying information selected and input by a user for example in a user ID input screen, not shown, of the presenting section 18 provided in the cell observation information processing installation, and a cell name, first culture tool identifying information and second culture tool identifying information designated and/or input by the user, for example via the tag selecting section 16 or the tag inputting section 21, in a search tag designating and/or inputting field 18d shown in FIG. 6 of the presenting section 18 provided in the cell observation information processing installation 1.

In the present embodiment, the user ID is used as the user identifying information for convenience, but the user identifying information is not limited to the user ID and may instead, for example, be a letter or a symbol that can distinguish a certain user from other users, such as the name or a nickname of the user.

In FIG. 3, the reference character 18a denotes a user ID display item, the reference character 18b denotes a date/time information display item, the reference character 18c denotes an observation information display field for cell image, activity data, and other pieces of information, and the reference character 18d denotes a search tag input field.

The user ID display item 18a is configured to display the user identifying information selected and input by the user in the user ID input screen. The date/time information display item 18b is configured to display the imaging date and time recorded as a log in a cell image in the observation information acquired via the observation information acquiring section 20. The observation information display field 18c is configured to display a cell image in the observation information acquired via the observation information acquiring section 20 and activity data, which indicate cell activity by the number of cells, cell confluency, cell morphology, cell viability or so, detected for example by performing image processing and image analysis on the cell image via an image processor and an image analyzer that are not shown.

The search tag designating and/or inputting field 18d is configured to allow the user to designate and/or input, for example, the cell identifying information including the cell name, the first culture tool identifying information, and the second culture tool identifying information as a part of the search tag via the tag selecting section 16 or the tag inputting section 21. The search tag designating and/or inputting field 18d is further configured to allow the user to designate and/or input, for example, the passage number, which is a part of the cell identifying information, as another part of the search tag via the tag selecting section 16 or the tag inputting section 21. The search tag designating and/or inputting field 18d may further be configured to allow the user to designate and/or input the user ID as the user identifying information via the tag selecting section 16 or the tag inputting section 21.

The presenting section 18 is further configured to display passage work presence/absence selecting buttons 18e and allow the user to select an order regarding whether or not the operation performed on the cells at the time of observation is passage work. The content of the selected order is acquired as a part of the search tag by the tag acquiring section 11.

The search tag designating and/or inputting field 18d in FIG. 6 may, of course, be configured to allow the user to designate and/or input, as other tags that the tag acquiring section 11 can acquire as a part of the search tag, desired subordinate-concept items in the user identifying information, the cell identifying information, the date/time information for identification of the date and time when the observation information was acquired, information on user' s work for cell maintenance, apparatus identifying information, information on a change in the activity data, and an image tag (for example, cell type, cell-level information indicating parent cell line or subculture, etc. under the category of cell identifying information) via the tag selecting section 16 or the tag inputting section 21.

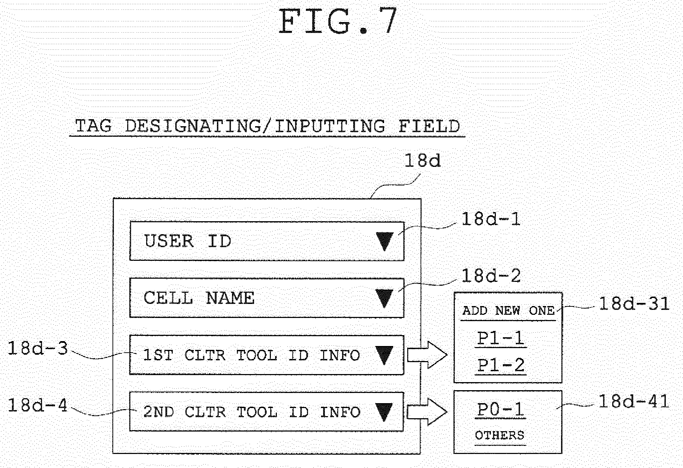

FIG. 7 is an explanatory diagram that schematically shows an example of key items in the search tag designating and/or inputting field 18d. In FIG. 7, the reference numeral 18d-1 denotes a user ID designating and/or inputting item, the reference numeral 18d-2 denotes a cell name designating and/or inputting item, the reference numeral 18d-3 denotes a first culture tool designating and/or inputting item, and the reference numeral 18d-4 denotes a second culture tool designating and/or inputting item. In the search tag designating and/or inputting field 18d in the example shown in FIG. 7, the user ID designating and/or inputting item 18d-1 is prepared so that a user ID can be designated and/or input. However, in a case where the tag acquiring section 11 acquires a user ID selected by the user in a user ID input screen that has been described above but is not shown, preparing the user ID designating and/or inputting item is not needed.

The tag acquiring section 11 searches through the archive section 13 using the user ID and the cell name designated and/or input by the user via the tag selecting section 16 or the tag inputting section 21 and retrieves search-tagged observation information having a largest passage number of cells cultured in the first culture tool among pieces of matched search-tagged observation information. The tag acquiring section 11 then causes the presenting section 18 to output, via the outputting section 19, the first culture tool identifying information included in the retrieved search-tagged observation information as possible first culture tool identifying information to be acquired as a part of the search tag. The presenting section 18 has a display field 18d-31, which displays a list of possible first culture tool identifying information output via the outputting section 19 along with a bar for new registration in the pulldown menu format, as shown in FIG. 7. In the example shown in FIG. 7, "P1-1" and "P1-2" are shown as bars each representing pieces of the possible first culture tool identifying information. Further, "add new one" is shown as a bar representing new registration.

In the present embodiment, the first culture tool identifying information is formed of the passage number of cells cultured in the first culture tool and identifying information specific to the first culture tool. For example, in "P1-1" shown as the bar representing a piece of first culture tool identifying information in the display field 18d-31, "P1" is an identification number for identifying the passage number, and "1" is an identification number for identifying the first culture tool. Further, in the present embodiment, the passage number is expressed by a numeral accompanied by a letter, "P" in the present embodiment, for convenience, but the passage number may, of course, be expressed only by a numeral.

It is configured so that, in the case where a bar of any piece of possible first culture tool identifying information is selected by the user in the display field 18d-31 via the tag selecting section 16, the tag acquiring section 11 automatically acquires, as a part the search tag, the second culture tool identifying information in the search-tagged observation information having the first culture tool identifying information selected by the user.

It is configured so that, in the case where the bar for new registration is selected by the user via the tag selecting section 16, the presenting section 18 accepts user's designation and/or input of a first culture tool in the first culture tool identifying information designating and/or inputting item 18d-3 via the tag selecting section 16 or the tag inputting section 21.

The tag acquiring section 11 then searches through the archive section 13 using the user ID and the cell name designated and/or input by the user via the tag selecting section 16 or the tag inputting section 21, and retrieves search-tagged observation information having passage presence/absenceinformationindicating passage work and having the latest observation information acquisition time point in the date/time information among the matched search-tagged observation information. The tag acquiring section 11 then causes the presenting section 18 to output, via the outputting section 19, in the form of a list, the first culture tool identifying information in the retrieved search-tagged observation information as possible second culture tool identifying information to be acquired as a part of the search tag. As shown in FIG. 7, the presenting section 18 has a display field 18d-41, which displays a list of pieces of the possible second culture tool identifying information output via the outputting section 19 along with a bar for designating other piece of second culture tool identifying information in the pulldown menu format. In the example shown in FIG. 7, "P0-1" is shown as a bar representing a piece of possible second culture tool identifying information.

In the present embodiment, a piece of the second culture tool identifying information is formed of a passage number of the cells cultured in the second culture tool and identification information specific to the second culture tool. For example, in "P0-1" shown as the bar representing a piece of the possible second culture tool identifying information in the display field 18d-41, "P0" is an identification number for identifying the passage number, and "1" is an identification number for identifying the second culture tool.

It is configured so that, in the case where any of the bars of the second culture tool identifying information is selected by the user in the display field 18d-41 via the tag selecting section 16, the tag acquiring section 11 acquires the second culture tool identifying information selected by the user as a part of the search tag.

It is configured so that, in the case where the bar for designating other second culture tool is selected, the presenting section 18 accepts user's designation/input of the second culture tool in the second culture tool identifying information designating/inputting item 18d-4 via the tag selecting section 16 or the tag inputting section 21.

The tag acquiring section 11 then acquires the second culture tool identifying information designated and/or input by the user via the tag selecting section 16 or the tag inputting section 21 as a part of the search tag.

In the example shown in FIG. 7, in preparation for acquisition of the first culture tool identifying information as the search tag, a list of possible first culture tool identifying information output via the outputting section 19 is displayed along with a bar for new registration in the display field 18d-31 in the pulldown menu format, and the user is prompted to select any of the first culture tool identifying information bars from the list of the possible first culture tool identifying information or the bar for new registration. The user may instead be prompted to directly designate and/or input first culture tool identifying information via the first culture tool identifying information designating and/or inputting item 18d-3.

In this case, the tag acquiring section 11 acquires the first culture tool identifying information designated and/or input by the user as a part of the search tag and searches through the archive section 13 using the user ID, the cell name, and the first culture tool identifying information designated and/or input by the user.

It is configured so that, if matched search-tagged observation information is present, the tag acquiring section automatically acquires, as second culture tool identifying information in the search tag, the second culture tool identifying information included in the matched search-tagged observation information.

On the other hand, if no matched search-tagged observation information is present, the tag acquiring section 11 further searches through the archive section 13 using the user ID and the cell name designated and/or input by the user, to retrieve search-tagged observation information having passage presence/absence information indicating passage work and having a latest observation information acquisition time point in the date/time information among matched search-tagged observation information in the new search. The tag acquiring section 11 then causes the presenting section 18 to output, via the outputting section 19, the first culture tool identifying information included in the retrieved search-tagged observation information as pieces of possible second culture tool identifying information to be acquired as a part of the search tag in the form of a list in the display field 18d-41 of the presenting section 18.

The configurations of the tag acquiring section 11 and the presenting section 18 in the case where any of the second culture tool identifying information bars is selected by the user in the display field 18d-41 via the tag selecting section 16 or in the case where the bar for designating another second culture tool is selected by the user have been described above.

The tag acquiring section 11 further acquires imaging date and time recorded as a log in a cell image in each piece of observation information acquired via the observation information acquiring section 20 as date/time information for identifying the date and time when the observation information was acquired. The tag acquiring section 11 is further configured to be capable of acquiring an image tag from the cell image in the observation information acquired via the observation information acquiring section 20.

The image tag is a tag for showing a cell image, in more detail, a small-sized image that can be displayed in an image selecting input screen presented in the presenting section 18 but not shown or information formed of an image and a name associated with the image integrated with each other.

The tag selecting section 16 is configured to allow the user to select a desired bar from the list of the bars displayed in the pulldown menu format in the display fields 18d-31, 18d-41 and the like in the presenting section 18.

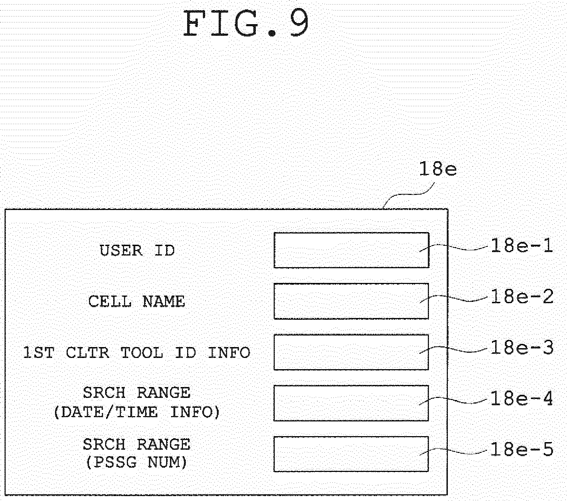

The tag inputting section 21 is configured to allow the user to manually input a desired tag using letter keys, numeral keys, symbol keys or another keys in each item for designation and/or input provided in the search tag designating and/or inputting field 18d or a search criterion designating and/or inputting field 18e, which will be described later and is shown in FIG. 9, displayed in the presenting section 18.

The outputting section 19 is configured to output information retrieved by the tag acquiring section 11 or the retrieving section 15, which will be described later, to the presenting section 18.

The observation information acquiring section 20 is configured to acquire observation information in response to an acquisition order at one time point. In more detail, the observation information acquiring section 20 operates when the user inputs an observation name and presses down an observation information acquisition order button in an image capture order screen provided in the installation 1 but not shown, causes an imaging device provided in the installation 1 but not shown to capture an image of a specimen containing cells located in an observation position, performs, for example, image processing and image analysis on the captured cell image via an image processor and an image analyzer that are not shown, to detect activity data indicating cell activity by the number of cells, cell confluency, cell morphology and/or cell viability, and temporarily stores, for example, the cell image, the activity data, and the observation name as one piece of observation information in a storage area that is not shown but is provided in the installation 1.

The storing section 12 is configured to assign a search tag acquired by the tag acquiring section 11 to each piece of observation information acquired by the observation information acquiring section 20 in response to an acquisition order at each time point and store search-tagged observation information to which the search tag is assigned in the archive section 13.

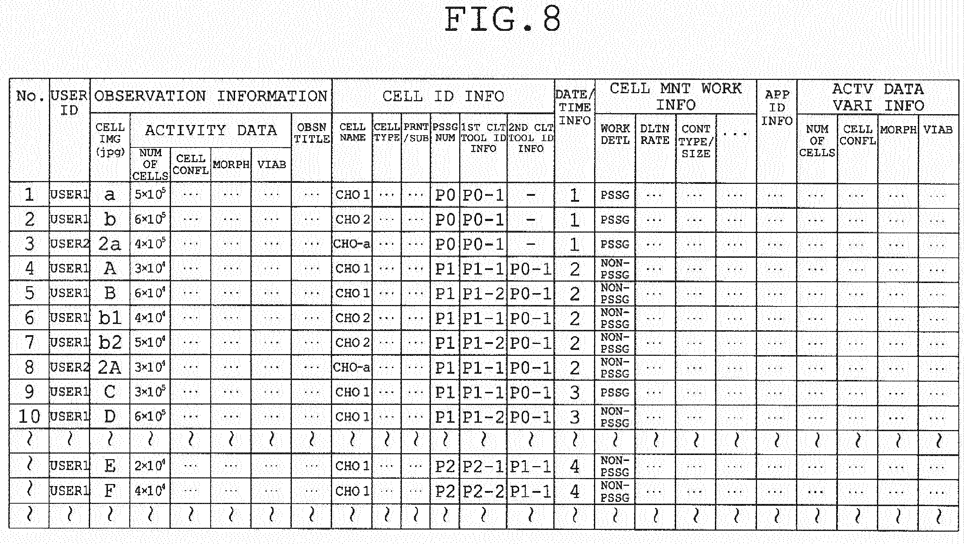

The archive section 13 has one or more search-tagged observation information records, as shown, for example, in FIG. 8. Each of the records is created in correspondence with operation of capturing an image of cells at each time point, and one piece of observation information corresponding to the operation of capturing an image of cells at each time point is related to the search tag for searching for observation information.

The search criterion acquiring section 14 is configured to acquire, as a search criterion, tags containing a cell name and first culture tool identifying information out of the search tag assigned to the search-tagged observation information.

To be specific, the search criterion acquiring section 14 is configured to acquire, as the search criterion, tags containing the following items: the user ID as the user identifying information; the cell name as the cell identifying information; and the first culture tool identifying information designated and/or input by the user via the tag selecting section 16 or the tag inputting section 21, for example, in the search criterion designating and/or inputting field 18e in the presenting section 18, such as that shown in FIG. 9, provided in the installation 1.

In FIG. 9, the reference numeral 18e-1 denotes a user ID designating and/or inputting item, the reference numeral 18e-2 denotes a cell name designating and/or inputting item, and the reference numeral 18e-3 denotes a first culture tool designating and/or inputting item. These items are configured as items mandatorily designated and/or input by the user.

Each of the designating and/or inputting items 18e-1 to 18e-3 may instead be configured to be selected and input by the user from several choices of information pieces displayed in the display field in the pulldown menu format that is not shown.

The search criterion designating field 18e may further be configured to allow the user to designate and/or input, as a range over which certain observation information is retrieved on the basis of a search criterion, a range of passage number and a range of date/time information on observation information acquisition time points. In FIG. 9, the reference numeral 18e-4 denotes an item for designating and/or inputting a range of date/time information, and the reference numeral 18e-5 denotes an item for designating and/or inputting a range of passage number. These items are configured to allow the user to select either of them and designate and/or input information to the selected item as required.

The date/time information range designating and/or inputting item 18e-4 is configured to prompt the user to designate and/or input date/time information on observation information acquisition time points at the start and the end of the search range. The date/time information range designating and/or inputting item 18e-4 may instead be configured to prompt the user to designate and/or input a retrospective period starting at the current observation information acquisition time point.

The passage number range designating and/or inputting item 18e-5 is configured to prompt the user to designate and/or input passage numbers at the start and the end of the search range. The passage number range designating and/or inputting item 18e-5 may instead be configured to prompt the user to designate and/or input a number of generations ascending the current passage number.

Further, the search criterion designating and/or inputting field 18e in FIG. 9 is merely an example showing only key items in the embodiment of the present invention for convenience. The search criterion designating and/or inputting field 18e may provide, as other tags for search criterion acquirable by the search criterion acquiring section 14, input items for any combination of desired subcategory items in the user identifying information, the cell identifying information, the date/time information for identification of the date and time when the observation information was acquired, the information on user's work for cell maintenance, the apparatus identifying information, the information on change in activity data, and the image tag (forexample, cell type, cell-level information indicating whether cells are of subculture or parent cell line, passage number etc. in the cell identifying information) .

The information on user's work for cell maintenance is, in more detail, identification information including at least one of the work details, the dilution ratio, and the type and size of the container.

The dilution ratio is the amount of thinning used when cells cultured in a culture container are thinned for passage or experiment.

The information on change in activity data is, for example, the amount of change (that is, rate of change) in activity data (confluency, number, and viability of cells, for example) between a plurality of time points and can be shown, for example, by the gradient of a graph.

The user ID designating and/or inputting item 18e-1 in the search criterion designating and/or inputting field 18e in the example shown in FIG. 9 may be configured to initially display the user ID selected and input by the user in the user ID input screen. In this case, an item for user ID display may be further provided in place of the user ID designating and/or inputting item 18e-1 to merely display the user ID selected and input by the user in the user ID input screen, so that user's operation of inputting user ID is omitted.

The retrieving section 15 is configured to search through the archive section 13 using the search criterion acquired by the search criterion acquiring section 14 and retrieve search-tagged observation information on cells having undergone passage of culture from a single cell line over a plurality of generations.

Tobe specific, the retrieving section 15 is configuredto repeat, on the basis of a predetermined retrieval range of observation information, the process of searching through the archive section 13 using the search criterion acquired by the search criterion acquiring section 14 and retrieving matched search-tagged observation information, then further re-searching through the archive section 13 using a search criterion in which the second culture tool identifying information provided in the retrieved search-tagged observation information replaces and serves as first culture tool identifying information in the search criterion, and retrieving matched search-tagged observation information.

The predetermined retrieval range of observation information is configured to be specified as follows, for example, in accordance with whether or not there is an input via the date/time information range designating and/or inputting item 18e-4 or the passage number range designating and/or inputting item 18e-5 in the search criterion designating and/or inputting field 18e:

For example, in the search criterion designating and/or inputting field 18e in FIG. 9, in a case where no designation and/or input is made via the date/time information range designating and/or inputting item 18e-4 or the passage number range designating and/or inputting item 18e-5, the predetermined retrieval range of observation information is so specified that "observation information is retrieved until last traceable data in the database is reached", that is, "until search-tagged observation information having no second culture tool identifying information recorded therein is retrieved".

The retrieving section 15 then repeats the process of re-searching through the archive section 13 by a search criterion in which the second culture tool identifying information included in the retrieved search-tagged observation information replaces and serves as first culture tool identifying information in the search criterion and retrieving matched search-tagged observation information until search-tagged observation information having no second culture tool identifying information is retrieved.

Instead, for example, in the search criterion designating and/or inputting field 18e in FIG. 9, in a case where designation and/or input is made via the date/time information range designating and/or inputting item 18e-4, the predetermined retrieval range of observation information is defined by "the range of date/time information designated and/or input to the date/time information range designating and/or inputting item 18e-4".

The retrieving section 15 then repeats the process of re-searching through the archive section 13 using a search criterion in which the second culture tool identifying information included in the retrieved search-tagged observation information replaces and serves as first culture tool identifying information in the search criterion and retrieving matched search-tagged observation information until retrieval of every search-tagged observation information including date/time information in the range of date/time information designated and/or input in the date/time information range designating and/or inputting item 18e-4 are completed.

Instead, for example, in the search criterion designating and/or inputting field 18e in FIG. 9, in a case where designation and/or input is made via the passage number range designating and/or inputting item 18e-5, the predetermined retrieval range of observation information is defined by "the range of passage number designated and/or input via the passage number range designating and/or inputting item 18e-5".

The retrieving section 15 then repeats the process of re-searching through the archive section 13 using a search criterion in which the second culture tool identifying information included in the retrieved search-tagged observation information replaces and serves as the first culture tool identifying information in the search criterion and retrieving matched search-tagged observation information until retrieval of every search-tagged observation information having a passage numbers within the range of passage number designated and/or input via the passage number range designating and/or inputting item 18e-5 are completed.

The cell observation information processing installation 1 includes, for example, a system login screen that is not shown. When the user inputs a system login ID and a password in the system login screen, the user ID input screen that is not shown is displayed. When the user inputs by selection his or her own user ID as the user identifying information in the user ID input screen via a user ID selecting/inputting section, the user ID is temporarily stored in a storage area that is not shown but is provided in the installation 1, and the image capture order screen that is not shown is displayed and allows the user to perform image capture order operation. The user ID input screen may be configured to also function as the system login screen so that the user inputs by selection the user ID and inputs the system login ID and a password.

Further, in the cell observation information processing system 10, the presenting section 18 is preferably provided with a display screen that is not shown but displays data including search-tagged observation information retrieved by the retrieving section 15 to the user.

A description will be made of a processing procedure of management of the condition of cells using the thus configured cell observation information processing system according to the first embodiment.

User's management of the condition of cultured cells is formed of the stage of storing observation information on the cultured cells in the archive section and the stage of searching for and retrieving desired observation information on the cells from the archive section and checking the condition of the cells.

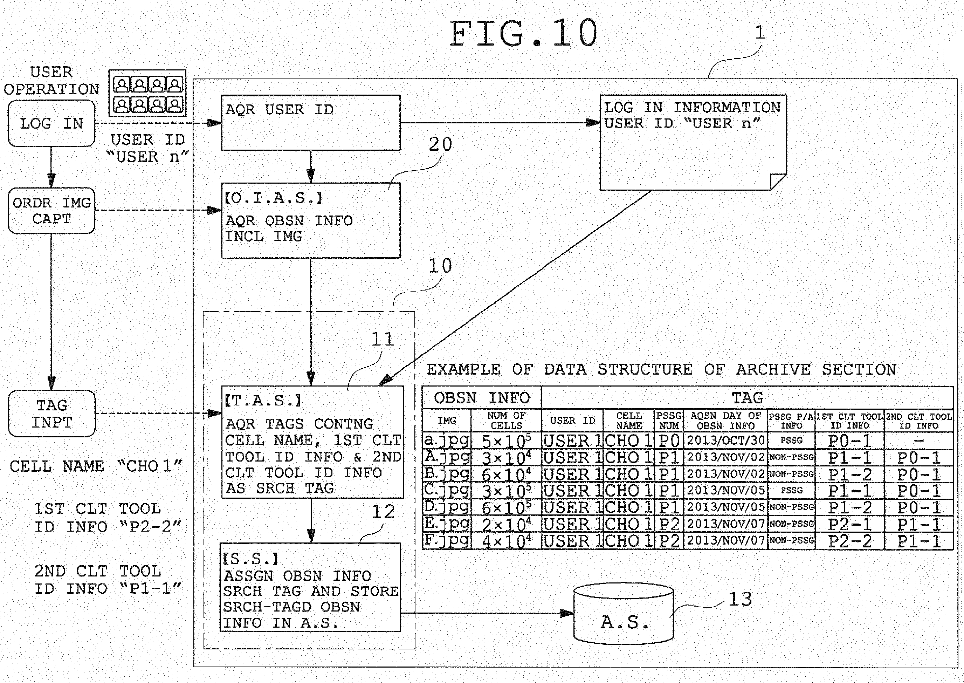

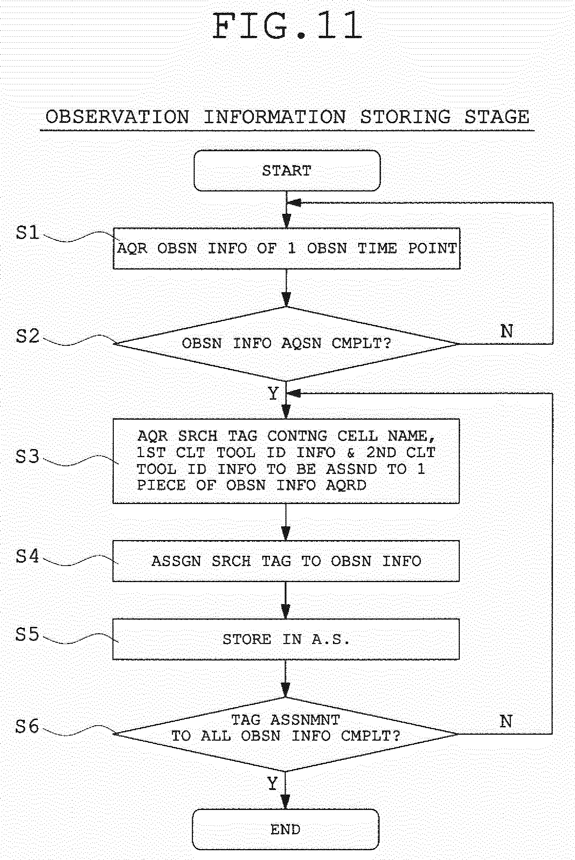

The processing procedure at the stage of storing observation information on the cultured cells in the archive section will first be schematically described with reference to FIGs. 10 and 11. In the example of the data structure of the archive section shown in FIG. 10, only a part of the data items is shown for convenience.

The user inputs the system login ID and the password in the system login screen that is not shown. The user ID input screen that is not shown is then displayed. The user then inputs by selection his or her own user ID ("user 1" in the example shown in FIG. 10). Consequently, the user ID is temporarily stored in the storage area in the installation 1, and the image capture order screen that is not shown is displayed, allowing the user to perform image capture ordering.

The user then inputs an observation name in the image capture order screen that is not shown and presses down an order button for ordering observation information acquisition. The observation information acquiring section 20 then causes the imaging device that is not shown to capture an image of a specimen containing cells located in an observation position and performs, for example, image processing and image analysis via an image processor and an image analyzer that are not shown on the captured cell image to detect the activity data representing cell activity, such as the number, confluency, morphology, and viability of the cells. The cell image, the activity data, and the observation name are then stored as one piece of observation information in a temporary storage area that is not shown but provided in the installation 1. Observation information at one time point is thus acquired (step S1). The acquisition of observation information can be repeatedmultiple times at different acquisition time points (step S2).

Upon completion of the input and/or observation image acquisition instruction made by the user in the image capture order screen and the observation information acquisition performed by the observation information acquiring section 20, the user designates and/or inputs, as a search tag to be assigned to the observation information, the user ID, the cell name, the first culture tool identifying information, and the second culture tool identifying information along, for example, with the cell-level information on the parent cell line or the new cell line and the passage number in the search tag designating and/or inputting field 18d, for example, via the respective designating and/or inputting/designating items and display screens. After the input is completed, the tag acquiring section 11 acquires, as a search tag, all tags including the cell name, the first culture tool identifying information, and the second culture tool identifying information designated and/or input by the user in the search tag designating and/or inputting field 18d (as for user ID tag, user ID designated and/or input by user in search tag designating and/or inputting field 18d or user ID selected and input by user in user ID selection screen) (step S3).

In the example shown in FIG. 11, after the observation information acquiring section 20 repeatedly acquires observation information multiple times, the presenting section is so switched that a tag is designated and/or input in the search tag designating and/or inputting field 18d and the tag acquiring section 11 acquires the search tag. Instead, whenever the observation information acquiring section 20 acquires observation information, the presenting section 18 may be so switched that a tag is designated and/or input in the search tag designating and/or inputting field 18d and the tag acquiring section 11 acquires the search tag. As for the timing at which the search tag is assigned, the presenting section 18 may be so switched that a tag is input in the search tag designating and/or inputting field 18d and the tag acquiring section 11 acquires the search tag in real time after the observation information acquiring section 20 acquires observation information, or the presenting section 18 maybe so switched that a tag is designated and/or input in the search tag designating and/or inputting field 18d and the tag acquiring section 11 acquires the search tag after fixed period after the observation information acquiring section 20 acquires observation information.

The storing section 12 then assigns the search tag acquired by the tag acquiring section 11 to the observation information acquired in response to the acquisition instruction at each time point (stepS4) and stores the search-tagged observation information to which the search tag is assigned in the archive section 13 (step S5).

Upon completion of the assignment of the search tag to the observation information at each time point and the storage of the search-tagged observation information in the archive section 13, the procedure at the stage of storing observation information on cultured cells in the archive section is completed (step S6).

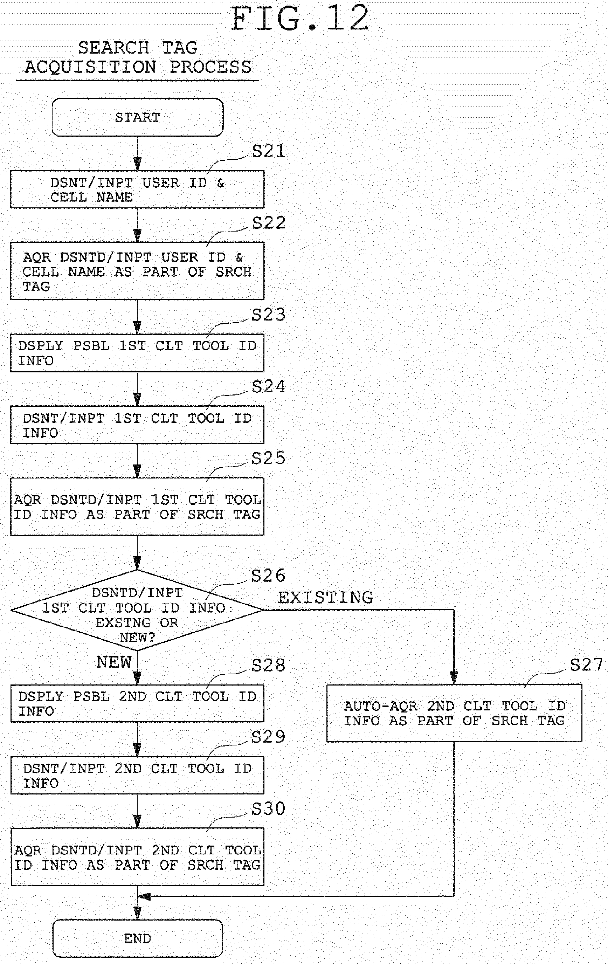

The procedure of acquiring essential tags in the search tag acquisition process (step S3) will now be further described. FIG. 12 is a flowchart that schematically shows an example of the procedure for acquiring essential tags in the search tag acquisition process shown in FIG. 11. In the description, the essential tags are assumed to be the user ID, the cell name, the first culture tool identifying information, and the second culture tool identifying information.

In the search tag acquisition process, the user designates and/or inputs the user ID and the cell name via the presenting section 18 (step S21). The tag acquiring section 11 then acquires, as a part of the search tag, the user ID and the cell name designated and/or input by the user (step S22). The tag acquiring section 11 then causes the presenting section 18 to present, in the form of a list, pieces of possible first culture tool identifying information to be acquired as a part of the search tag (step S23). The user designates and/or inputs desired first culture tool identifying information out of the possibles displayed by the presenting section 18 in the form of a list (step S24). The tag acquiring section 11 then acquires, as a part of the search tag, the first culture tool identifying information designated and/or input by the user (step S25).

In a case where the first culture tool identifying information designated and/or input by the user is existing information as first culture tool identifying information in search-tagged observation information including the same user ID and cell name stored in the archive section 13, the tag acquiring section 11 acquires, as a part of the search tag, the second culture tool identifying information included in the search-tagged observation information including the first culture tool identifying information (step S26, step S27).

On the other hand, in a case where the first culture tool identifying information designated and/or input by the user is new information as the first culture tool identifying information in search-tagged observation information including the same user ID and cell name stored in the archive section 13, the tag acquiring section 11 causes the presenting section 18 to display, in the form of a list, pieces of possible second culture tool identifying information to be acquired as a part of the search tag (step S28). The user designates and/or inputs desired second culture tool identifying information out of the pieces of possible second culture tool identifying information displayed in the presenting section 18 (step S29). The tag acquiring section 11 then acquires, as a part of the search tag, the second culture tool identifying information designated and/or input by the user (step S30).

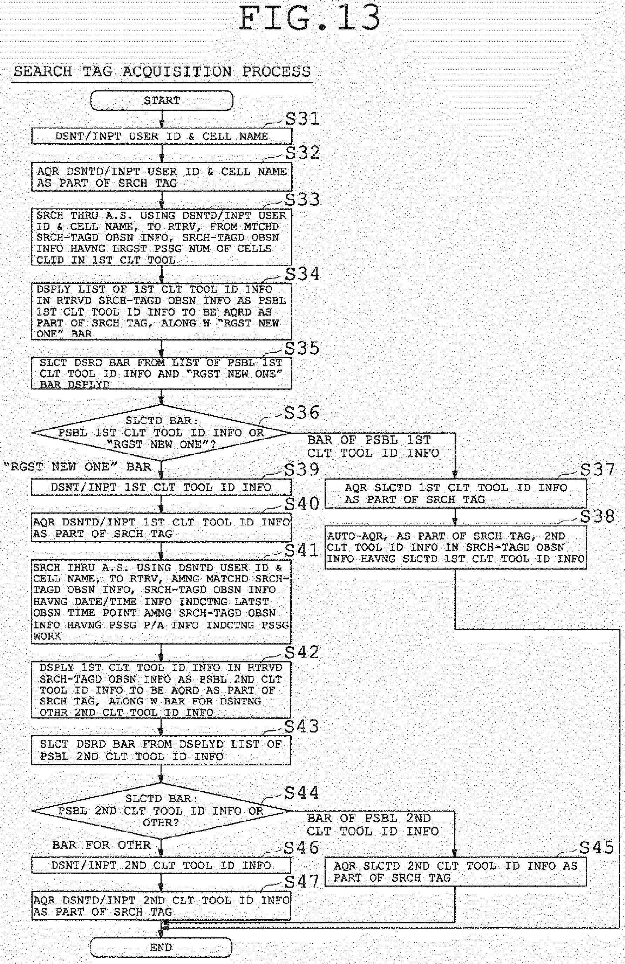

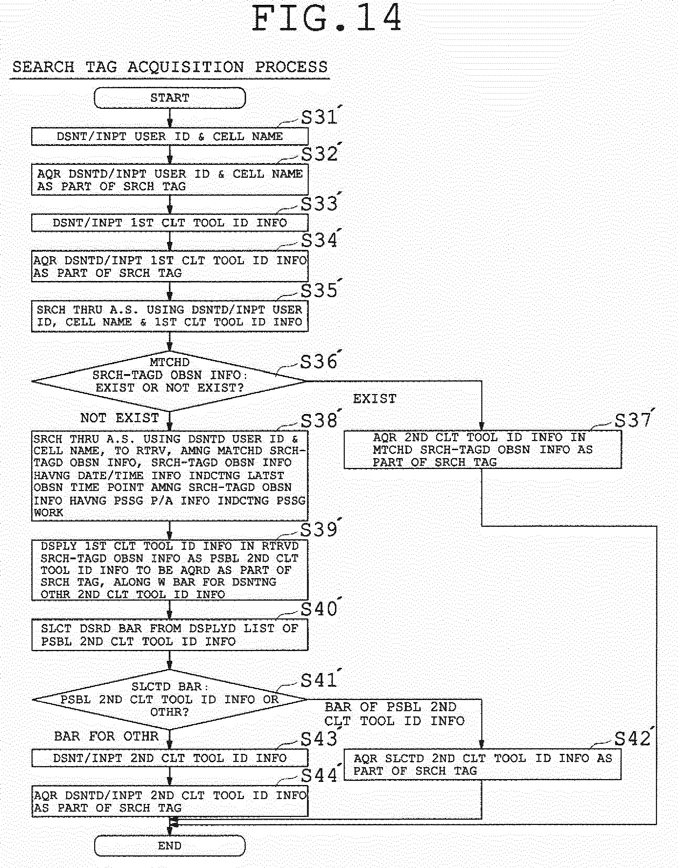

Further, details of processing procedure for acquiring the essential tags shown in FIG. 12 will be described with reference to FIGs. 13 and 14.

FIG. 13 is a flowchart that shows an example of the processing procedure as the details of the procedure for acquiring essential tags shown in FIG. 12, and FIG. 14 is a flowchart that shows a variation of the processing procedure in FIG. 13.

The processing procedure in the example shown in FIG. 13 will first be described.

In the processing procedure in the example shown in FIG. 13, the user designates and/or inputs the user ID via the user ID designating and/or inputting item 18d-1 in the search tag designating and/or inputting field 18d shown in FIG. 7 or the user ID designating screen that is not shown and designates and/or inputs the cell name via the cell name designating and/or inputting item 18d-2 in the search tag designating and/or inputting field 18d (step S31). The tag acquiring section 11 then acquires, as a part of the search tag, the user ID and the cell name designated and/or input by the user (step S32). The tag acquiring section 11 then searches through the archive section 13 using the user ID and the cell name designated and/or input by the user and retrieves search-tagged observation information having the largest passage number of the cells cultured in the first culture tool among pieces of matched search-tagged observation information (step S33). The tag acquiring section 11 then causes the presenting section 18 to output, via the outputting section 19, in the form of a list, the first culture tool identifying information included in the retrieved search-tagged observation information as pieces of possible first culture tool identifying information to be acquired as a part of the search tag. The presenting section 18 displays the list of the possible first culture tool identifying information output via the outputting section 19, along with the new registration bar, in the display screen 18d-31 in the pulldown menu format (step S34).

The user then selects a desired bar from the list of the possible first culture tool identifying information and the new registration bar displayed in the display screen 18d-31 (step S35).

In a case where the user selects any of the bars representing the pieces of possible first culture tool identifying information, the tag acquiring section 11 acquires, as a part of the search tag, the first culture tool identifying information selected by the user (steps S36, S37). The tag acquiring section 11 then automatically acquires, as a part of the search tag, the second culture tool identifying information included in the search-tagged observation information including the first culture tool identifying information selected by the user (step S38).

On the other hand, in a case where the user selects the new registration bar, the presenting section 18 accepts user's designation and/or input of the first culture tool identifying information via the first culture tool identifying information designating and/or inputting item 18d-3. The user then designates and/or inputs the first culture tool identifying information via the first culture tool identifying information designating and/or inputting item 18d-3 in the search tag designating and/or inputting field 18d (steps S36, S39). The tag acquiring section 11 then acquires, as a part of the search tag, the first culture tool identifying information designated and/or input by the user (step S40).

The tag acquiring section 11 then searches through the archive section 13 using the user ID and the cell name designated and/or input by the user and retrieves search-tagged observation information having passage presence/absence information that indicates passage work and having the latest observation information acquisition time point in the date/time information among the pieces of matched search-tagged observation information (step S41). The tag acquiring section 11 then causes the presenting section 18 to output, via the outputting section 19, in the form of a list, first culture tool identifying information included in the retrieved search-tagged observation information as pieces of possible second culture tool identifying information to be acquired as a part of the search tag. The presenting section 18 displays the list of the pieces of possible second culture tool identifying information output via the outputting section 19, along with a bar for designating other piece of second culture tool identifying information, in the display screen 18d-41 in the pulldown menu format (step S42).

The user then selects a desired bar from the list of the pieces of possible second culture tool identifying information and the bar for designating other piece of second culture tool identifying information displayed in the display screen 18d-41 (step S43).

In a case where the user selects any of the bars representing the second culture tool identifying information, the tag acquiring section 11 acquires, as a part of the search tag, the second culture tool identifying information selected by the user (steps S44 and S45).

On the other hand, in a case where the user selects the bar for designating other second culture tool, the presenting section 18 accepts user's designation and/or input of the second culture tool via the second culture tool identifying information designating and/or inputting item 18d-4. The user then designates and/or inputs a piece of second culture tool identifying information via the second culture tool identifying information designating and/or inputting item 18d-4 in the search tag designating and/or inputting field 18d (steps S44 and S46). The tag acquiring section 11 then acquires, as a part of the search tag, the second culture tool identifying information designated and/or input by the user (step S47).

The processing procedure in the variation shown in FIG. 14 will next be described.

The processing procedure in which the user designates and/or inputs the user ID and the cell name (step S31') and the processing procedure in which the tag acquiring section 11 acquires the user ID and the cell name as a part of the search tag (step S32') are roughly the same as the processing procedures in the example shown in FIG. 13.

The user then designates and/or inputs the first culture tool identifying information via the first culture tool identifying information designating and/or inputting item 18d-3 in the search tag designating and/or inputting field 18d shown in FIG. 7 (step S33'). The tag acquiring section 11 then acquires, as a part of the search tag, the first culture tool identifying information designated and/or input by the user (step S34'). The tag acquiring section 11 then searches through the archive section 13 using the user ID, the cell name, and the first culture tool identifying information designated and/or input by the user (step S35').

In a case where matched search-tagged observation information is present as a result of the search, the tag acquiring section 11 automatically acquires, as a part of the search tag, the second culture tool identifying information included in the search-tagged observation information (steps S36' and S37').

On the other hand, in a case where no matched search-tagged observation information is present as a result of the search, the tag acquiring section 11 further searches through the archive section 13 using the user ID and the cell name designated and/or input by the user and retrieves search-tagged observation information having passage presence/absence information that indicates passage work and having the latest observation information acquisition time point in the date/time information among pieces of matched search-tagged observation information (steps S36' and S38'). The tag acquiring section 11 then causes the presenting section 18 to output, via the outputting section 19, in the form of a list, first culture tool identifying information provided in the retrieved search-tagged observation information having the first culture tool identifying information, as pieces of possible second culture tool identifying information to be acquired as a part of the search tag. The presenting section 18 displays the list of the pieces of possible second culture tool identifying information output via the outputting section 19 along with a bar for designating other second culture tool in the display field 18d-41 in the pulldown menu format (step S39').

The subsequent processing procedure after user's selection of a desired bar (step S40') is roughly the same as the processing procedure in the example in FIG. 10.

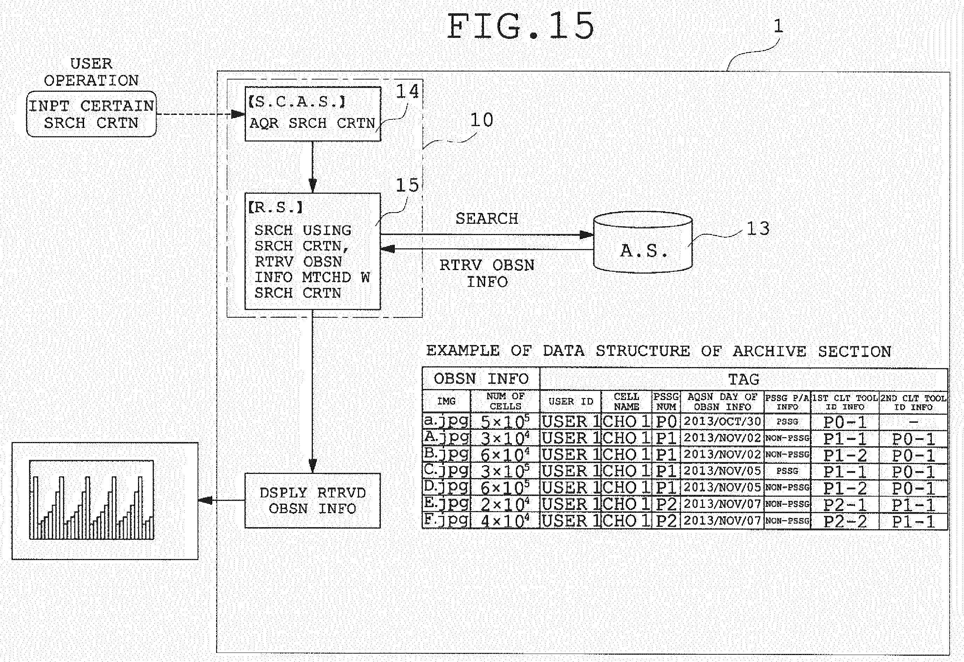

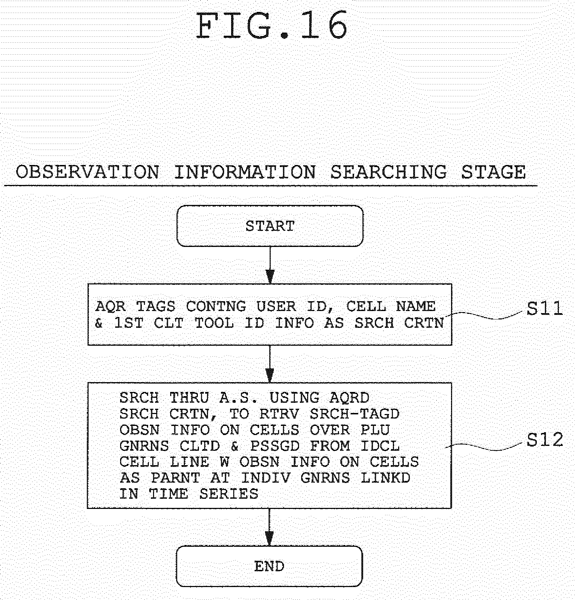

The processing procedure at the stage of searching for and/or retrieving desired cell observation information from the archive section and checking the condition of cells will next be described with reference to FIGs. 15 and 16. In the example of the data structure of the archive section shown in FIG. 15, only a part of the data items is shown for convenience.

The user designates tags containing the user ID, the cell name, and the first culture tool identifying information as a search criterion in the search criterion designating and/or inputting field 18e, such as that shown in FIG. 9. Upon completion of the input, the search criterion acquiring section 14 acquires, as the search criterion, the information input by the user in the search criterion designating field (step S11). Regarding the user ID in the search criterion designating and/or inputting field 18e in FIG. 9, it may be configured to initially display the user ID input by selection by the user in the user ID input screen, as described above, so that user's input is omitted.

The retrieving section 15 then searches through the archive section 13 using the search criterion acquired by the search criterion acquiring section 14 and retrieves, from the archive section 13, search-tagged observation information on cells having undergone passage of culture from a single cell line over a plurality of generations (step S12). The procedure for searching for and/or retrieving desired cell observation information from the archive section is thus completed.

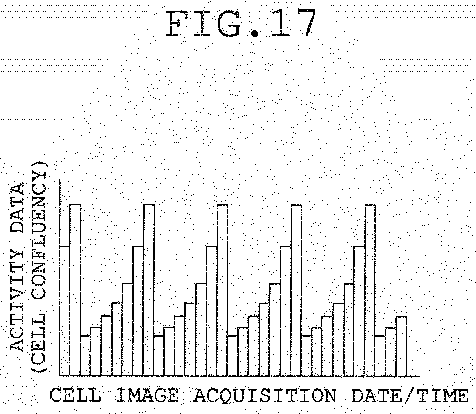

The data including the search-tagged observation information retrieved by the retrieving section 15 is displayed in a display screen, which is not shown, in a display mode according to the search criterion acquired by the search criterion acquiring section 14. The user checks the condition of the cells by observing the data displayed on the display screen in a display mode such as that shown in FIG. 17. The procedure at the stage of searching for and/or retrieving desired cell observation information from the archive section 13 and checking the condition of cells is thus completed.

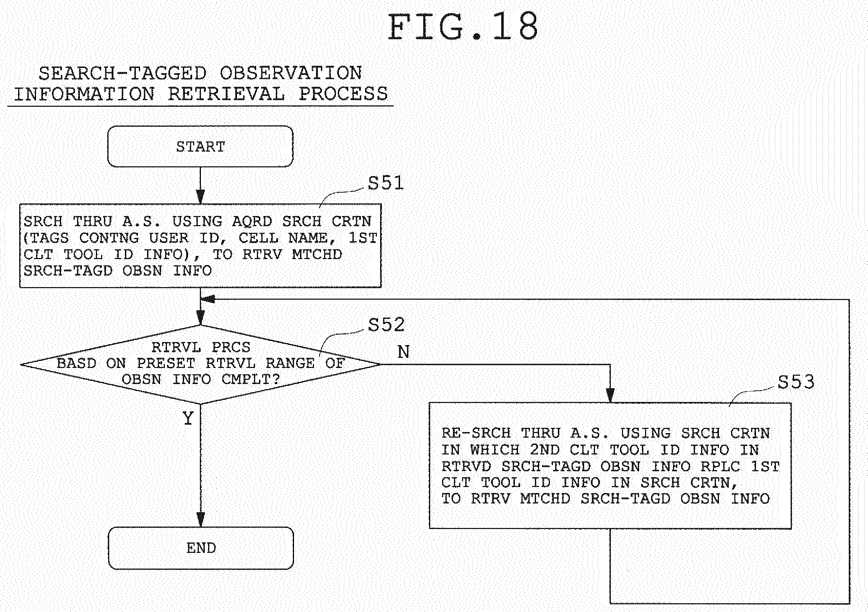

The process of retrieving search-tagged observation information on cells having undergone passage of culture from a single cell line over a plurality of generations (step S12), which is carried out by the retrieving section 15, will be further described. FIG. 18 is a flowchart that shows an example of the processing procedure for the retrieval process by the retrieving section shown in FIG. 16 of the search-tagged observation information on cells having undergone passage of culture from a single cell line over a plurality of generations.

In the process of acquiring search-tagged observation information, the retrieving section 15 searches through the archive section 13 using the search criterion (tags containing user ID, cell name, and first culture tool identifying information) acquired by the search criterion acquiring section 14 and retrieves matched search-tagged observation information (step S51). The retrieving section 15 further repeats, on the basis of a predetermined retrieval range of observation information, the process of re-searching through the archive section 13 using a search criterion in which the second culture tool identifying information included in the retrieved search-tagged observation information replaces and serves as first culture tool identifying information in the search criterion and retrieving matched search-tagged observation information (steps S52 and S53).

The predetermined retrieval range of observation information is defined, in accordance with the items designated and/or input by the user in the search criterion designating and/or inputting field 18e shown in FIG. 9, to be any of "a range back to last traceable data in the database", "a range of date/time information on observation information acquisition time point", and "a range of passage number".

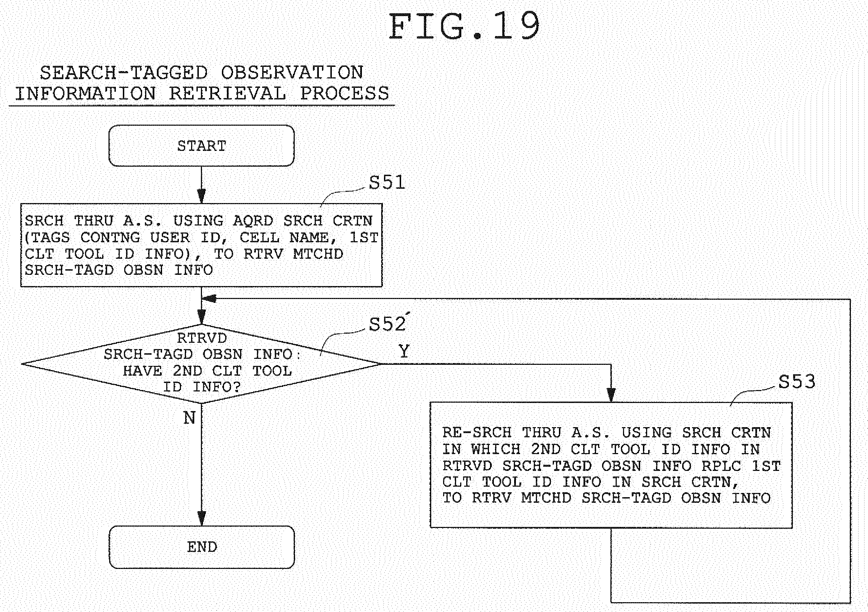

The predetermined retrieval range of observation information is defined to be "a range back to last traceable data in the database" in a case where no user's designation and/or input is made via the date/time information range designating and/or inputting item 18e-4 or the passage number range designating and/or inputting item 18e-5 in the search criterion designating and/or inputting field 18e shown in FIG. 9. In this case, the retrieving section 15 repeats the process of re-searching through the archive section 13 using a search criterion in which the second culture tool identifying information included in the retrieved search-tagged observation information replaces and serves as first culture tool identifying information in the search criterion and retrieving matched search-tagged observation information until search-tagged observation information having no second culture tool identifying information recorded therein is retrieved (steps S52' and S53'), as shown in FIG. 19.

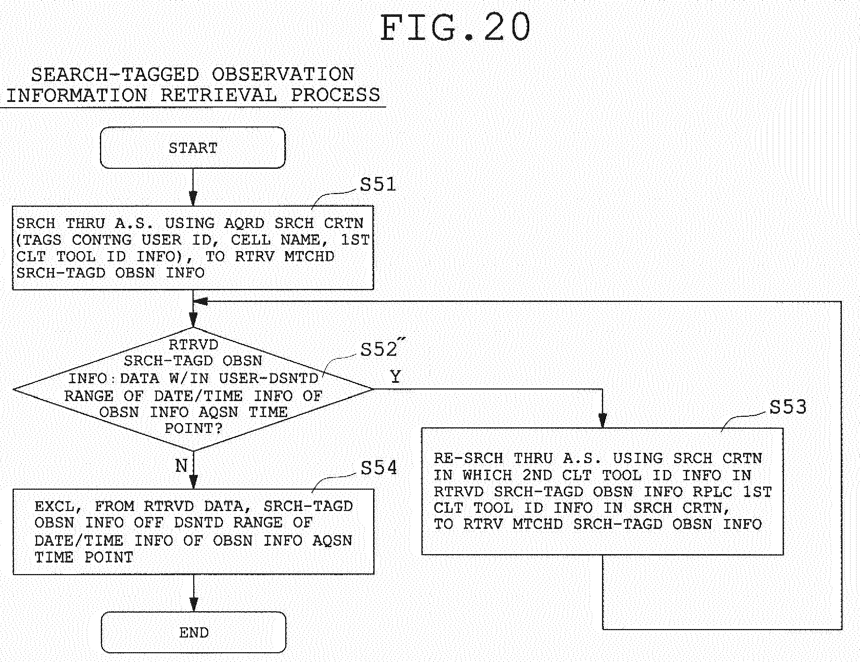

The predetermined retrieval range of observation information is defined by "a range of date/time information on observation information acquisition time point" in a case where user's designation and/or input is made via the date/time information range designating and/or inputting item 18e-4 in the search criterion designating and/or inputting field 18e shown in FIG. 9. In this case, the retrieving section 15 repeats the process of re-searching through the archive section 13 using a search criterion in which the second culture tool identifying information included in the retrieved search-tagged observation information replaces and serves as first culture tool identifying information in the search criterion and retrieving matched search-tagged observation information until retrieval of every search-tagged observation information having date/time information within the range of date/time information designated and/or input via the date/time information range designating and/or inputting item 18e-4 is completed (steps S52", S53, and S54), as shown in FIG. 20.

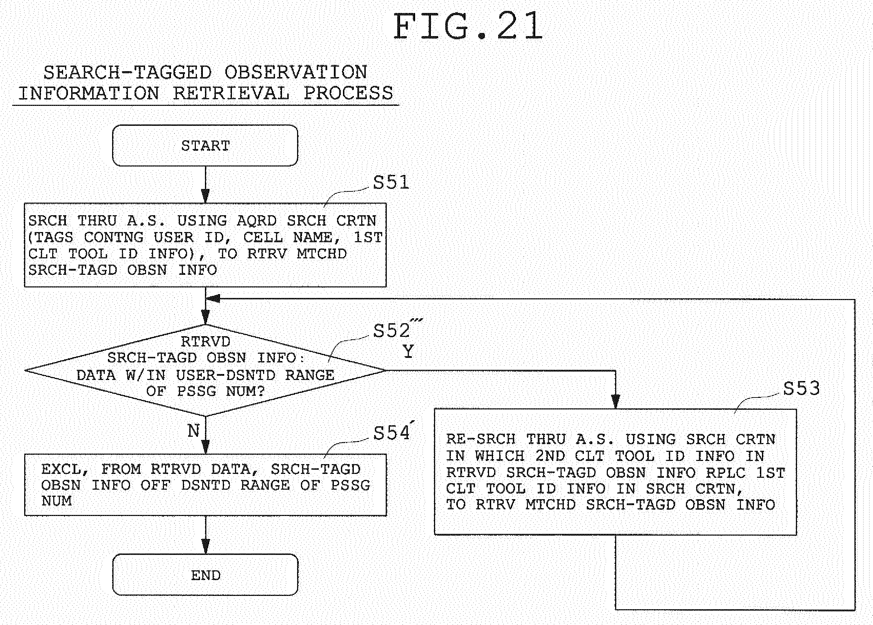

The predetermined retrieval range of observation information is defined by "a range of passage number" in a case where designation and/or input is made via the passage number range designating and/or inputting item 18e-5 in the search criterion designating and/or inputting field 18e shown in FIG. 9. In this case, the retrieving section 15 repeats the process of re-searching through the archive section 13 using a search criterion in which the second culture tool identifying information included in the retrieved search-tagged observation information replaces and serves as first culture tool identifying information in the search criterion and retrieving matched search-tagged observation information until retrieval of every search-tagged observation information having a passage number within the range of passage number designated and/or input via the passage number range designating and/or inputting item 18e-5 is completed (steps S52"', S53, and S54'), as shown in FIG. 21.

Assuming that search-tagged observation information in which the user ID is "user 1" and the cell name is "CHO1" and which has the data structure shown in FIG. 10 or 15 is stored in the archive section 13, a specific description will be made of retrieval of search-tagged observation information on cells so cultured over a plurality of generations that observation information on cells cultured in a first culture container having first culture tool identifying information "P2-2" links with observation information on cells that serve as passage sources in the generations on the time series.

The user designates and/or inputs "user 1" via the user ID designating and/or inputting item 18e-1, "CHO1" via the cell name designating and/or inputting item 18e-2, and "P2-2" via the first culture tool designating and/or inputting item 18e-3 in the search criterion designating and/or inputting field 18e in FIG. 9. In the description, it is assumed that no designation and/or input is made via the date/time information range designating and/or inputting item 18e-4 or the passage number range designating and/or inputting item 18e-5 (that is, the predetermined retrieval range of observation information is specified to be "the range to last traceable data in the database") for convenience.

The search criterion acquiring section 14 acquires tags containing the user ID "user 1", the cell name "CHO1", and the first culture tool identifying information "P2-2" as the search criterion (step S11).

The retrieving section 15 searches through the archive section 13 using the search criterion (tags containing user ID "user 1", cell name "CHO1", and first culture tool identifying information "P2-2") acquired by the search criterion acquiring section 14 and retrieves every matched search-tagged observation information (search-tagged observation information labeled with reference character R7 in FIG. 22(a)) (step S51).

The search-tagged observation information labeled with reference character R7 has "P1-1" recorded therein as the second culture tool identifying information.

The retrieving section 15 then re-searches through the archive section 13 using a search criterion in which the second culture tool identifying information "P1-1" included in the search-tagged observation information labeled with reference character R7 replaces and serves as first culture tool identifying information in the search criterion, to retrieve every matched search-tagged observation information (search-tagged observation information labeled with reference characters R2 and R4 in FIG. 22 (a)) (steps S52 and S53).

The search-tagged observation information labeled with reference characters R2 and R4 has "P0-1" recorded therein as the second culture tool identifying information.

The retrieving section 15 then re-searches through the archive section 13 using a search criterion in which the second culture tool identifying information "P0-1" included in the search-tagged observation information labeled with reference character R2 replaces and serves as first culture tool identifying information in the search criterion, to retrieve every matched search-tagged observation information (search-tagged observation information labeled with reference character R1 in FIG. 22(a)) (steps S52 and S53).

The search-tagged observation information labeled with reference character R1 has no second culture tool identifying information recorded therein.

The retrieving section 15 then terminates the process of retrieving search-tagged observation information (steps S52 and S53).

As a result, among the pieces of search-tagged observation information labeled with R1 to R7 in FIG. 22 (a) and having the same user ID "user 1" and cell name "CHO1" recorded therein, the retrieving section 15 retrieves the following pieces of search-tagged observation information (1) to (3) labeled with the star mark:
(1) search-tagged observation information (R7) having the first culture tool identifying information "P2-2" recorded therein;
(2) search-tagged observation information (R2, R4) which is associated with cells serving as the passage source of the cells having the search-tagged observation information (R7) and in which the second culture tool identifying information "P1-1" recorded in the search-tagged observation information (R7) is recorded as the first culture tool identifying information; and
(3) search-tagged observation information (R1) which is associated with cells that serve as the passage source of the cells having the search-tagged observation information (R2, R4) and in which the second culture tool identifying information "P0-1" recorded in the search-tagged observation information (R2, R4) is recorded as the first culture tool identifying information.

The user can therefore retrieve only data on search-tagged observation information on cells having undergone passage of culture from a single cell line over a plurality of generations, for example, as indicated with the chain double-dashed line in FIG. 22 (b), only by designating and/or inputting first culture tool identifying information as a tag other than the user ID or the cell name, for example, in the search criterion designating and/or inputting field 18e, such as that shown in FIG. 9.

According to the cell observation information processing system 10 of the first embodiment, the tag acquiring section 11 acquires tags containing a cell name, first culture tool identifying information, and second culture tool identifying information as a search tag, and the storing section 12 stores search-tagged observation information to which the search tag is assigned in the archive section 13, whereby the first culture tool identifying information contained in the search tag in the search-tagged observation information stored in the archive section 13 allows culture tools having the same cell name and the same passage number to be distinguished from one another, and the second culture tool identifying information allows observation information on passage-destination cells and observation information on passage-source cells to be related to each other over a plurality of generations. The search criterion acquiring section 14 then acquires tags containing the cell name and the first culture tool identifying information as the search criterion, and the retrieving section 15 searches through the archive section 13 using the search criterion acquired by the search criterion acquiring section 14 and retrieves search-tagged observation information on cells having undergone passage of culture from a single cell line over a plurality of generations, whereby the user can grasp observation information on specific-line cells over generations even in passage of culture of the one-to-many passage type.

Further, according to the cell observation information processing system 10 of the first embodiment, the retrieving section 15 repeats, on the basis of a predetermined retrieval range of observation information, the process of searching through the archive section 13 using the search criterion acquired by the search criterion acquiring section 14, retrieving matched search-tagged observation information, then further re-searching the archive section 13 using a search criterion in which the second culture tool identifying information included in the retrieved search-tagged observation information replaces and serves as first culture tool identifying information in the search criterion and retrieving matched search-tagged observation information, whereby search-tagged observation information on cells having undergone passage of culture from a single cell line over a plurality of generations canbe retrieved by the retrieving section 15.

Further, according to the cell observation information processing system 10 of the first embodiment, the predetermined retrieval range of observation information is configured so that observation information is retrieved until search-tagged observation information having no second culture tool identifying information recorded therein is retrieved, whereby search-tagged observation information on cells over a plurality of generations can be retrieved back to the oldest passage source generation traceable in the database.

Further, according to the cell observation information processing system 10 of the first embodiment, a search tag further includes date/time information at an observation information acquisition time point and the predetermined retrieval range of observation information is configured to be the date/time information range determined by the observation information acquisition time point designated by user's operation, or a search tag contains the passage number of cells cultured in the first culture tool and the predetermined retrieval range of observation information is configured to be the passage number range designated by user's operation, whereby search-tagged observation information on cells over a plurality of generations can be retrieved over a range necessary for the user.

Further, according to the cell observation information processing system 10 of the first embodiment, the first culture tool identifying information is formed of the passage number of cells cultured in the first culture tool and identification information specific to the first culture tool, whereby the configuration described above in conjunction with the configuration in which the second culture tool identifying information is formed of the passage number of cells cultured in the second culture tool and identification information specific to the second culture tool eliminates the need to subtract 1 from the current passage number and separately set the resultant passage number as a part of the search criterion used when the retrieving section 15 searches through the archive section 13 in order to allow the retrieving section 15 to retrieve search-tagged observation information one generation before but allows retrieval of search-tagged observation information on cells having a passage number one generation before only by replacing the first culture tool identifying information in the search criterion with the value of the second culture tool identifying information included in the retrieved data.

In the cell observation information processing system 10 according to the first embodiment, the first culture tool identifying information may instead, of course, be configured not to include the passage number of the cells cultured in the first culture tool.

Further, according to the cell observation information processing system 10 of the first embodiment, since the second culture tool identifying information is formed of the passage number of cells cultured in the second culture tool and identification information specific to the second culture tool, the configuration eliminates the need to subtract 1 from the current passage number and separately set the resultant passage number as a part of the search criterion used when the retrieving section 15 searches through the archive section 13 in order to allow the retrieving section 15 to retrieve search-tagged observation information one generation before but allows the value of the passage number included in the second culture tool identifying information included in the retrieved data to be set as a part of the search criterion. Search-tagged observation information on cells having a passage number one generation before can therefore be retrieved by replacing the first culture tool identifying information in the search criterion with the value of the identification information specific to the second culture tool included in the second culture tool identifying information included in the retrieved data.

In the cell observation information processing system 10 according to the first embodiment, the second culture tool identifying information may instead, of course, be configured not to include the passage number of the cells cultured in the second culture tool.

Further, according to the cell observation information processing system 10 of the first embodiment, a search tag further includes a passage number of cells cultured in the first culture tool, and the tag acquiring section 11 searches through the archive section using a cell name designated by user's operation, retrieves search-tagged observation information having the largest passage number of the cells cultured in the first culture tool among pieces of matched search-tagged observation information, outputs, to the presenting section 18, first culture tool identifying information included in the retrieved search-tagged observation information in the form of a list as pieces of possible first culture tool identifying information to be acquired as a part of the search tag, acquires, as a part of the search tag, the first culture tool identifying information selected by user's operation from the pieces of possible first culture tool identifying information output to the presenting section 18 in the form of a list, and automatically acquires, as a part of the search tag, the second culture tool identifying information included in the search-tagged observation information including the first culture tool identifying information selected by user's operation, whereby user' s operation of typing keys to input the first culture tool identifying information and the second culture tool identifying information can be omitted, and an error of key typing can also be eliminated. As a result, the efficiency of search tag acquisition can be improved, and the time taken to check the condition of the cells can be shortened. In particular, in a situation where the condition of the cells is checked with the cells being taken out of an incubator, the time taken to check the condition of the cells is shortened, whereby the amount of damage on the cells can be reduced.

Further, according to the cell observation information processing system 10 of the first embodiment, the tag acquiring section 11 acquires, as a part of the search tag, the first culture tool identifying information designated by user's operation, searches through the archive section 13 using a cell name and the first culture tool identifying information designated by user's operation, and if matched search-tagged observation information is present, automatically acquires, as a part of the search tag, the second culture tool identifying information included in the search-tagged observation information, whereby user's operation of typing keys to input the second culture tool identifying information can be omitted, and an error of key typing can also be eliminated. Further, since operation of typing keys to input the second culture tool identifying information is omitted, the efficiency of search tag acquisition is improved accordingly, whereby the time taken to check the condition of the cells can be shortened.

Further, according to the cell observation information processing system 10 of the first embodiment, a search tag contains the passage presence/absence information representing whether or not operation performed on first cells is passage operation and the date/time information on an observation information acquisition time point, and if new culture tool identifying information is designated by user' s operation as the first culture tool identifying information to be acquired as a part of the search tag, the tag acquiring section 11 acquires, as a part of the search tag, the first culture tool identifying information designated by user's operation, searches through the archive section 13 using a cell name designated by user' s operation, retrieves search-tagged observation information having passage presence/absence information indicating passage work and having the latest observation information acquisition time point in the date/time information amongmatched search-tagged observation information, outputs, to the presenting section 18, the first culture tool identifying information included in the retrieved search-tagged observation information in the form of a list as pieces of possible second culture tool identifying information to be acquired as a part of the search tag, and acquires, from the pieces of possible second culture tool identifying information output to the presenting section 18 in the form of a list, the second culture tool identifying information selected by user's operation as a part of the search tag, whereby user's operation of typing keys to input the second culture tool identifying information can be omitted, and an error of key typing can also be eliminated. Further, since operation of typing keys to input the second culture tool identifying information is omitted, the efficiency of search tag acquisition is improved accordingly, whereby the time taken to check the condition of the cells can be shortened.

Further, according to the cell observation information processing system 10 of the first embodiment, a search tag includes the passage presence/absence information indicating whether or not operation performed on cells cultured in the first culture tool is passage work and date/time information on observation information acquisition time point, and the tag acquiring section 11 acquires, as a part of the search tag, the first culture tool identifying information designated by user's operation, searches through the archive section 13 using a cell name and the first culture tool identifying information designated by user's operation, further searches, if no matched search-tagged observation information is present, through the archive section 13 using the cell name designated by user's operation, retrieves search-tagged observation information having passage presence/absence information indicating passage work and having the latest observation information acquisition time point in the date/time information among pieces of matched search-tagged observation information, outputs, to the presenting section 18, the first culture tool identifying information included in the retrieved search-tagged observation information in the form of a list as pieces of possible second culture tool identifying information to be acquired as a part of the search tag, and acquires, among the pieces of possible second culture tool identifying information output to the presenting section 18 in the form of a list, a piece of second culture tool identifying information selected by user's operation as a part of the search tag, whereby user's operation of typing keys to input the second culture tool identifying information can be omitted, and an error of key typing can also be eliminated. Further, since operation of typing keys to input the second culture tool identifying information is omitted, the efficiency of search tag acquisition is improved accordingly, whereby the time taken to check the condition of cells can be shortened. Second embodiment

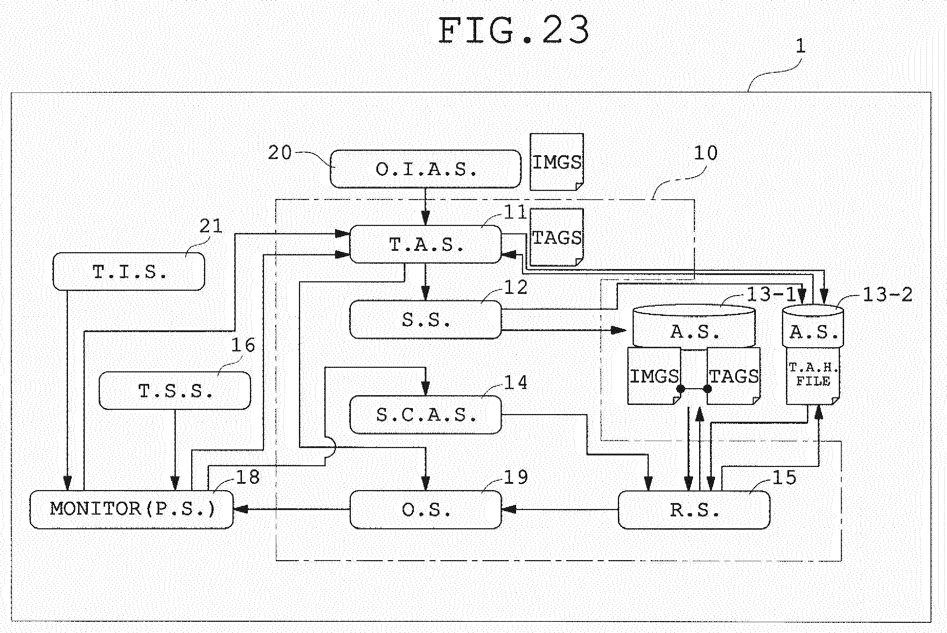

FIG. 23 is an explanatory diagram showing the configuration of a cell observation information processing system according to a second embodiment of the present invention. FIG. 21 is an explanatory diagram showing an example of the data structure of tag acquisition history information stored in the archive section in the cell observation information processing system.

In the cell observation information processing system 10 according to the second invention, the storing section 12 is configured to store search-tagged observation information, such as that shown in FIG. 8, in a first archive section 13-1 and relate information on a history of acquisition of a tag acquired as a search tag by the tag acquiring section 11 to, for example, the user ID and store the resultant information in a second archive section 13-2, which is a file separate from the first archive section 13-1. The archive section includes the first archive section 13-1 and the second archive section 13-2. The first archive section 13-1 is formed of a database file. The second archive section 13-2 is formed, for example, of a system log file.

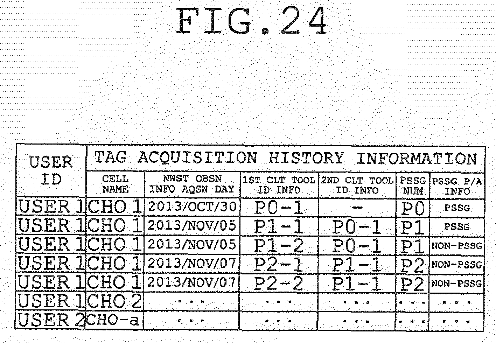

The information on tag acquisition history is formed, for example, of a cell name, the latest observation information acquisition date, first culture tool identifying information, and second culture tool identifying information acquired by the tag acquiring section 11, as shown in FIG. 24.

The second archive section 13-2 stores the latest observation information acquisition date, passage number, and passage presence/absence information corresponding to the same user ID, cell name, first culture tool identifying information, and second culture tool identifying information as information on the latest tag acquisition history updated as required at the time point when search-tagged observation information is stored in the first archive section 13-1.

The tag acquiring section 11 searches through the second archive section 13-2 using a user ID and a cell name designated and/or input by the user via the tag selecting section 16 or the tag inputting section 21 in the search tag designating and/or inputting field 18d and retrieves tag acquisition history information having the largest passage number of cells cultured in a first culture tool among pieces of matched tag acquisition history information. The tag acquiring section 11 then causes the presenting section 18 to output, via the outputting section 19, first culture tool identifying information included in the retrieved tag acquisition history information in the form of a list as pieces of possible first culture tool identifying information to be acquired as a part of the search tag.

In a case where the user selects any of the bars representing the pieces of possible first culture tool identifying information in the display field 18d-31 via the tag selecting section 16, the tag acquiring section 11 automatically acquires, as a part of the search tag, the second culture tool identifying information included in the tag acquisition history information having the first culture tool identifying information selected by the user.

In a case where the user selects the new registration bar via the tag selecting section 16, the presenting section 18 accepts user' s designation and/or input of the first culture tool via the tag selecting section 16 or the tag inputting section 21 via the first culture tool identifying information designating and/or inputting item 18d-3.

The tag acquiring section 11 then searches through the second archive section 13-2 using the user ID and the cell name designated and/or input by the user via the tag selecting section 16 or the tag inputting section 21 and retrieves tag acquisition history information having passage presence/absence information indicating passage work and having the latest observation information acquisition time point in the date/time information among pieces of matched tag acquisition history information. The tag acquiring section 11 then causes the presenting section 18 to output, via the outputting section 19, the first culture tool identifying information included in the retrieved tag acquisition history information in the formof a list as pieces of possible second culture tool identifying information to be acquired as a part of the search tag.

To acquire first culture tool identifying information as a search tag, the list of the pieces of possible first culture tool identifying information output via the outputting section 19 is displayed along with the bar for new registration in the display field 18d-31 in the pulldown menu format, and the user is prompted to select any of the bars representing the first culture tool identifying information from the list of the possible first culture tool identifying information or the bar for new registration. The user may instead be prompted to directly designate and/or input first culture tool identifying information via the first culture tool identifying information designating and/or inputting item 18d-3.

In this case, the tag acquiring section 11 acquires, as a part of the search tag, the first culture tool identifying information designated and/or input by the user and searches through the second archive section 13-2 using the user ID, the cell name, and the first culture tool identifying information designated and/or input by the user.

In a case where matched tag acquisition history information is present, the tag acquiring section 11 automatically acquires the second culture tool identifying information included in the tag acquisition history information as the second culture tool identifying information in a search tag.

On the other hand, in a case where no matched search-tagged observation information is present, the tag acquiring section 11 further searches through the second archive section 13-2 using the user ID and the cell name designated and/or input by the user and retrieves tag acquisition history information having passage presence/absence information indicating passage work and having the latest observation information acquisition time point in the date/time information among pieces of matched tag acquisition history information. The tag acquiring section 11 then outputs, via the outputting section 19, in the form of a list, the first culture tool identifying information included in the retrieved tag acquisition history information as pieces of possible second culture tool identifying information to be acquired as a part of the search tag in the display field 18d-41 of the presenting section 18.

The configurations of the tag acquiring section 11 and the presenting section 18 in a case where any of the bars representing second culture tool identifying information is selected by the user via the display field 18d-41 and via the tag selecting section 16 or in a case where a bar for designating another second culture tool is selected have been described above.

The retrieving section 15 repeats, on the basis of a predetermined retrieval range of acquisition history information, the process of searching through the second archive section 13-2 using the search criterion acquired by the search criterion acquiring section 14, retrieving matched tag acquisition history information, then further newly creating a search criterion in which the second culture tool identifying information included in the retrieved tag acquisition history information replaces and serves as first culture tool identifying information in the search criterion, re-searching through the second archive section 13-2 using the created search criterion, and retrieving matched tag acquisition history information. The retrieving section 15 then sequentially searches through the first archive section 13-1 using all the search criteria used to retrieve the tag acquisition history information on the basis of the predetermined retrieval range of acquisition history information and retrieves matched search-tagged observation information.

The predetermined retrieval range of tag acquisition history information is configured to be defined, for example, as follows in accordance with the content of an input via the date/time information range designating and/or inputting item 18e-4 or the passage number range designating and/or inputting item 18e-5 in the search criterion designating and/or inputting field 18e.

For example, in the search criterion designating and/or inputting field 18e in FIG. 9, in a case where no designation and/or input is made via the date/time information range designating and/or inputting item 18e-4 or the passage number range designating and/or inputting item 18e-5, the predetermined retrieval range of tag acquisition history information is so defined that "until last traceable data in the database of a tag acquisition history information file is reached", that is, "until tag acquisition history information having no second culture tool identifying information recorded therein is retrieved".

The retrieving section 15 then repeats the process of newly creating a search criterion in which the second culture tool identifying information included in the retrieved tag acquisition history information replaces and serves as first culture tool identifying information in the search criterion, re-searching through the second archive section 13-2 using the created search criterion, and retrieving matched tag acquisition history information until tag acquisition history information including no second culture tool identifying information is retrieved.

Further, for example, in the search criterion designating and/or inputting field 18e in FIG. 9, in a case where designation and/or input is made via the date/time information range designating and/or inputting item 18e-4, the predetermined retrieval range of tag acquisition history information is defined by "a range of date/time information designated and/or input via the date/time information range designating and/or inputting item 18e-4".

The retrieving section 15 then repeats the process of newly creating a search criterion in which the first culture tool identifying information in the search criterion is replaced with the second culture tool identifying information included in the retrieved tag acquisition history information, re-searching the second archive section 13-2 using the created search criterion, and retrieving matched tag acquisition history information until retrieval of every piece of tag acquisition history information including date/time information within the range of date/time information designated and/or input via the date/time information range designating and/or inputting item 18e-4 is completed.

Further, for example, in the search criterion designating and/or inputting field 18e in FIG. 9, in a case where designation and/or input is made via the passage number range designating and/or inputting item 18e-5, the predetermined retrieval range of tag acquisition history information is defined by "a range of passage number designated and/or input via the passage number range designating and/or inputting item 18e-5".

The retrieving section 15 then repeats the process of newly creating a search criterion in which the second culture tool identifying information included in the retrieved tag acquisition history information replaces and serves as first culture tool identifying information in the search criterion, searching through the second archive section 13-2 using the created search criterion, and retrieving matched tag acquisition history information until every piece of tag acquisition history information having a passage number within the range of passage number designated and/or input in the passage number range designating and/or inputting item 18e-5 is completed.

The other configurations are roughly the same as those of the cell observation information processing system 10 according to the first embodiment.

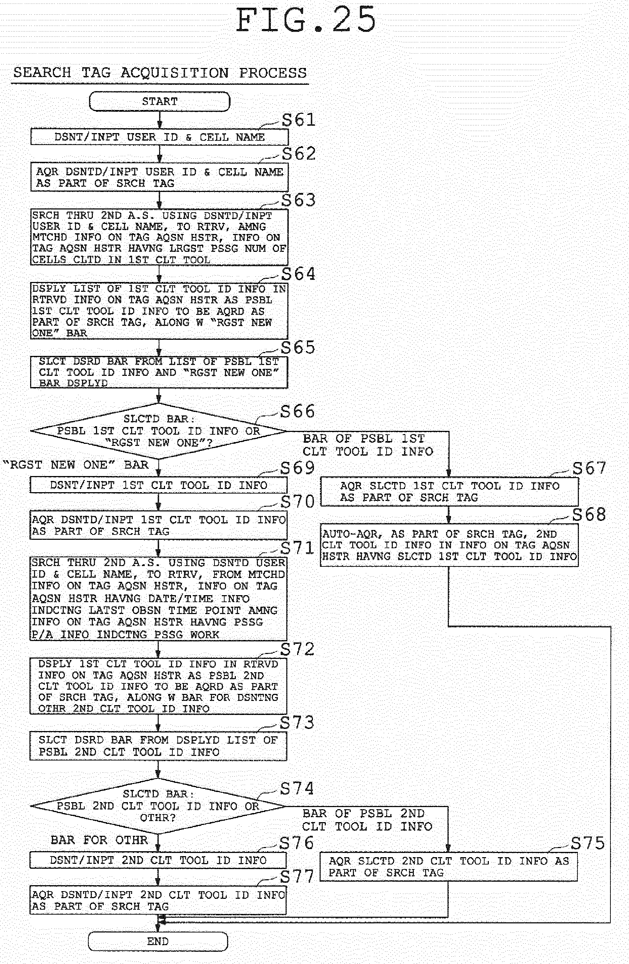

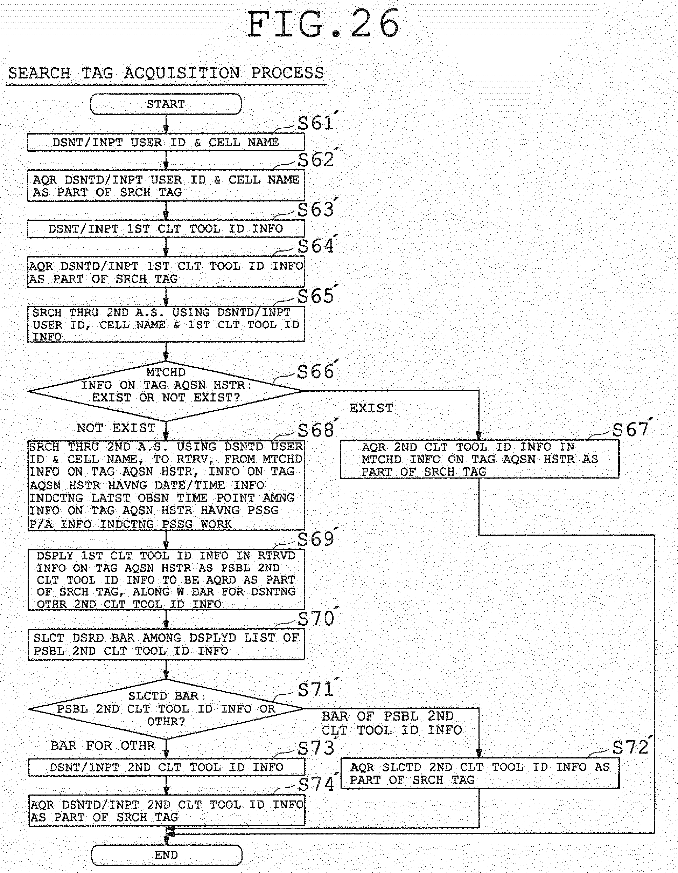

In the thus configured cell observation information processing system according to the second embodiment, the detail of the processing procedure for acquiring the essential tags in the search tag acquisition process (step S3 in FIG. 11) at the stage of storing observation information on cultured cells in the archive section differs from the processing procedure in the cell observation information processing system according to the first embodiment. The different processing procedure will be described below with reference to FIGs. 25 and 26.

FIG. 25 is a flowchart that shows an example of the processing procedure as the detail of the procedure for acquiring essential tags in the search tag acquisition process in the processing procedure at the stage of storing observation information on cells using the cell observation information processing system shown in FIG. 23, and FIG. 26 is a flowchart that shows a variation of the processing procedure in FIG. 25.

### first be described.

The processing procedure in which a user ID and a cell name are designated and/or input (step S61) and the processing procedure in which the tag acquiring section 11 acquires the user ID and the cell name as a part of the search tag are roughly the same as the processing procedure in the example shown in FIG. 13 in the first embodiment.

The tag acquiring section 11 then searches through the second archive section 13-2 using the user ID and the cell name designated and/or input in the form of a tag by the user and retrieves tag acquisition history information having passage presence/absence information indicating passage work and having the latest observation information acquisition time point in the date/time information among pieces of matched tag acquisition history information (step S63). The tag acquiring section 11 then causes the presenting section 18 to output, via the outputting section 19, the first culture tool identifying information included in the retrieved tag acquisition history information in the form of a list as pieces of possible second culture tool identifying information to be acquired as a part of the search tag. The presenting section 18 displays the list of the pieces of possible first culture tool identifying information output via the outputting section 19 along with the new registration bar in the display field 18d-31 in the pulldown menu format (step S64).

The user then selects a desired bar from the list of the pieces of possible first culture tool identifying information and the bar for new registration displayed in the display field 18d-31 (step S65) .

In a case where the user selects any of the bars representing the pieces of possible first culture tool identifying information, the tag acquiring section 11 acquires, as a part of the search tag, the first culture tool identifying information selected by the user (steps S66, S67). The tag acquiring section 11 then automatically acquires, as a part of the search tag, the second culture tool identifying information included in the tag acquisition history information including the first culture tool identifying information selected by the user (step S68).

On the other hand, in a case where the user selects the bar for new registration, the processing procedure in which the presenting section 18 accepts user's designation and/or input of first culture tool identifying information via the first culture tool identifying information designating and/or inputting item 18d-3 of the presenting section 18, the processing procedure in which the user designates and/or inputs first culture tool identifying information (steps S66, S69), and the processing procedure in which the tag acquiring section 11 acquires the first culture tool identifying information as a part of the search tag (step S70) is roughly the same as the processing procedures in the example shown in FIG. 13 in the first embodiment.

The tag acquiring section 11 then searches through the second archive section 13-2 using the user ID and the cell name designated and/or input by the user and retrieves tag acquisition history information having passage presence/absence information indicating passage work and having the latest observation information acquisition time point in the date/time information among pieces of matched tag acquisition history information (step S71). The tag acquiring section 11 then causes the presenting section 18 to output, via the outputting section 19, first culture tool identifying information included in the retrieved tag acquisition history information in the form of a list as pieces of possible second culture tool identifying information to be acquired as a part of the search tag. The presenting section 18 displays the list of the pieces of possible second culture tool identifying information output via the outputting section 19 along with the bar for designating other piece of second culture tool identifying information in the display field 18d-41 in the pulldown menu format (step S72).

The processing procedures of user's selection of a desired bar (step S73) and thereafter are roughly the same as the processing procedures in the example shown in FIG. 13 in the first embodiment.

The processing procedure in the variation shown in FIG. 26 will next be described.

The processing procedure in which the user designates and/or inputs a user ID and a cell name (step S61') to the processing procedure in which the tag acquiring section 11 acquires the first culture tool identifying information as a part of the search tag (step S64') are roughly the same as the processing procedures in the example shown in FIG. 14 in the first embodiment.

The tag acquiring section 11 then searches through the second archive section 13-2 using the user ID, the cell name, and the first culture tool identifying information designated and/or input by the user (step S65').

In a case where matched search-tagged observation information is present, the tag acquiring section 11 automatically acquires, as a part of the search tag, the second culture tool identifying information included in the tag acquisition history information (steps S66' and S67').

On the other hand, in a case where no matched search-tagged observation information is present, the tag acquiring section 11 further searches through the second archive section 13-2 using the user ID and the cell name designated and/or input by the user and retrieves tag acquisition history information having passage presence/absence information indicating passage work and having the latest observation information acquisition time point in the date/time information among pieces of matched tag acquisition history information (steps S66' and S68'). The tag acquiring section 11 then causes the presenting section 18 to output, via the outputting section 19, in the form of a list, the first culture tool identifying information included in the retrieved tag acquisition history information having first culture tool identifying information, as pieces of possible second culture tool identifying information to be acquired as a part of the search tag. The presenting section 18 displays a list of the pieces of possible second culture tool identifying information output via the outputting section 19 along with the bar for designating another second culture tool in the display field 18d-41 in the pulldown menu format (step S69').

The processing procedures of user's selection of a desired bar (step S70') and thereafter are roughly the same as the processing procedures in the example shown in FIG. 14 in the first embodiment.

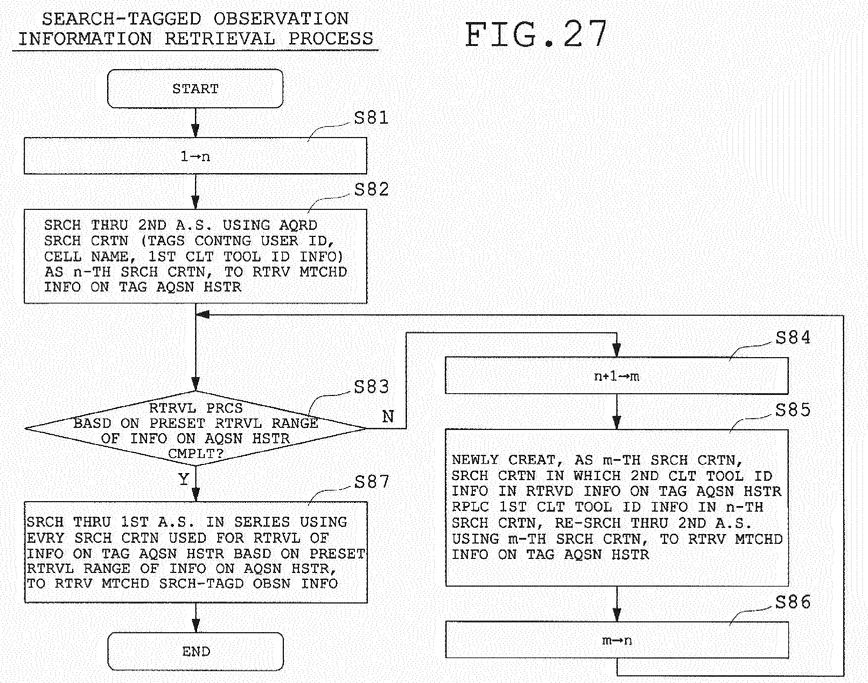

In the cell observation information processing system according to the second embodiment, the processing procedure of the search-tagged observation information retrieving process (step S12 in FIG. 16) at the stage of searching for and/or retrieving observation information on desired cells from the archive section and checking the condition of the cells differs from the processing procedure in the cell observation information processing system according to the first embodiment. The different processing procedure will be described below with reference to FIGs. 27 to 30.

FIG. 27 is a flowchart that shows an example of the processing procedure for retrieval process of the search-tagged observation information on cells having undergone passage of culture from a single cell line over a plurality of generations, at the stage of searching for and/or retrieving observation information on desired cells from the archive section using the cell observation in format ion processing system in FIG. 23.

In the process of acquiring search-tagged observation information, the retrieving section 15 searches through the second archive section 13-2 using the search criterion (tags containing user ID, cell name, and first culture tool identifying information) acquired by the search criterion acquiring section 14 and retrieves matched tag acquisition history information (steps S81 and S82). The retrieving section 15 further repeats, on the basis of a predetermined retrieval range of tag acquisition history information, the process of newly creating a search criterion in which the second culture tool identifying information included in the retrieved tag acquisition history information replaces and serves as first culture tool identifying information in the search criterion, searching through the second archive section 13-2 using the created search criterion, and retrieving matched tag acquisition history information (steps S83 to S86).

The predetermined retrieval range of tag acquisition history information is defined, in accordance with items designated and/or input by the user in the search criterion designating and/or inputting field 18e shown in FIG. 9, to be any of "a range back to last traceable data in the database of a tag acquisition history information file", "a range of date/time information on observation information acquisition time point", and "range of passage number".

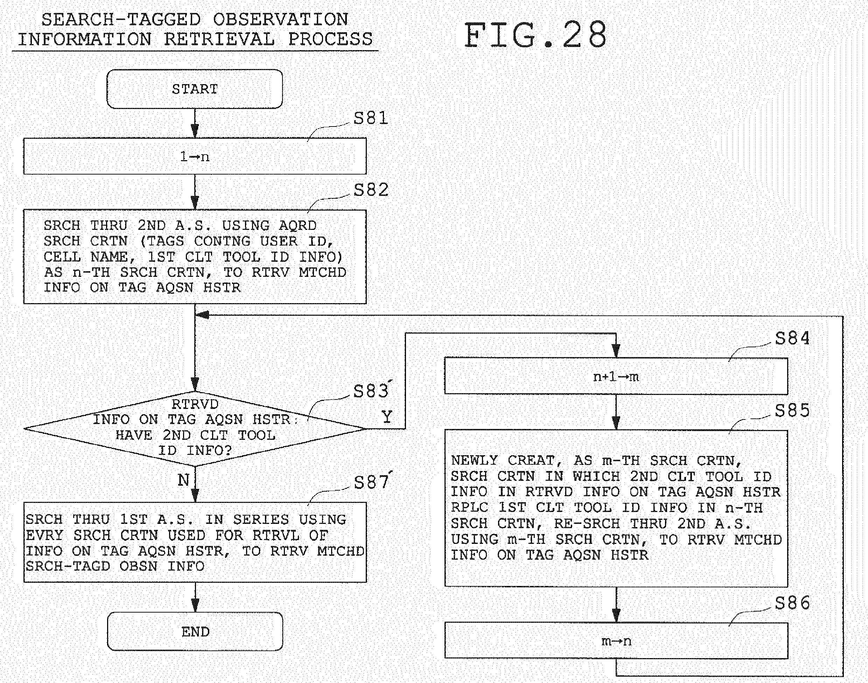

The predetermined retrieval range of tag acquisition history information is defined to be "a range back to last traceable data in the database of a tag acquisition history information file" in a case where no user's designation and/or input is made via the date/time information range designating and/or inputting item 18e-4 or the passage number range designating and/or inputting item 18e-5 in the search criterion designating and/or inputting field 18e shown in FIG. 9. In this case, the retrieving section 15 repeats the process of newly creating a search criterion in which the second culture tool identifying information included in the retrieved tag acquisition history information replaces and serves as first culture tool identifying information in the search criterion, searching through the second archive section 13-2 using the created search criterion, and retrieving matched tag acquisition history information until tag acquisition history information having no second culture tool identifying information recorded therein is retrieved (steps S83' to S86), as shown in FIG. 28.

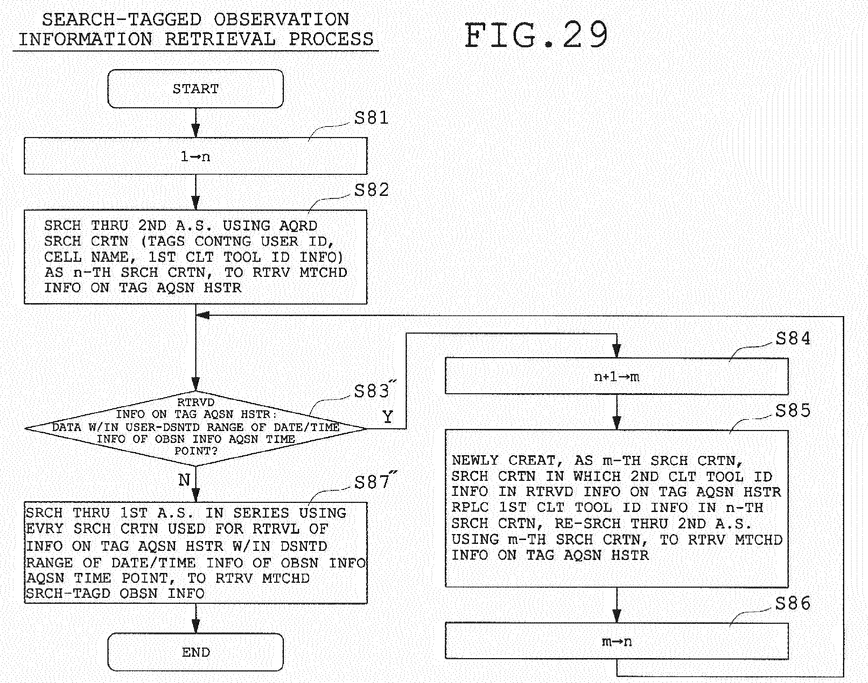

The predetermined retrieval range of tag acquisition history information is defined to be "a range of date/time information on observation information acquisition time point" in a case where user's designation and/or input is made via the date/time information range designating and/or inputting item 18e-4 in the search criterion designating and/or inputting field 18e shown in FIG. 9. In this case, the retrieving section 15 repeats the process of newly creating a search criterion in which the second culture tool identifying information included in the retrieved tag acquisition history information replaces and serves as first culture tool identifying information in the search criterion, searching through the second archive section 13-2 using the created search criterion, and retrieving matched tag acquisition history information until retrieval of every piece of tag acquisition history information having date/time information within the range of date/time information designated and/or input via the date/time information range designating and/or inputting item 18e-4 is completed (steps S83" to S86), as shown in FIG. 29.

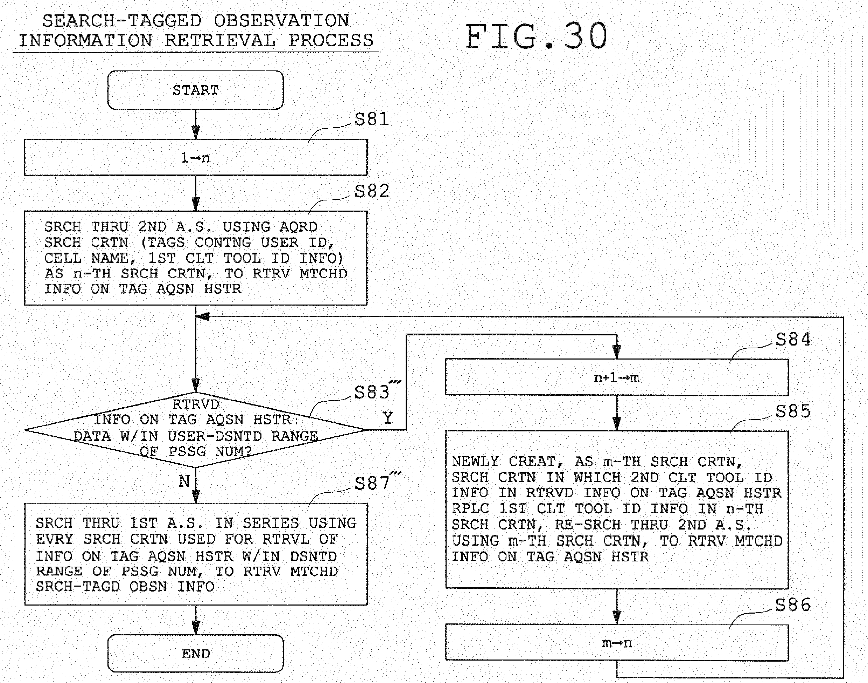

The predetermined retrieval range of tag acquisition history information is defined by "a range of passage number" in a case where designation and/or input is made via the passage number range designating and/or inputting item 18e-5 in the search criterion designating and/or inputting field 18e shown in FIG. 9. In this case, the retrieving section 15 repeats the process of newly creating a search criterion in which the second culture tool identifying information included in the retrieved tag acquisition history information replaces and serves as first culture tool identifying information in the search criterion, re-searching through the second archive section 13-2 using the created search criterion, and retrieving matched tag acquisition history information until retrieval of every piece of tag acquisition history information having a passage number within the range of passage number designated and/or input via the passage number range designating and/or inputting item 18e-5 is completed (steps S83"' to S86), as shown in FIG. 30.

The retrieving section 15 then sequentially searches through the first archive section 13-1 using all the search criteria used to retrieve the tag acquisition history information on the basis of the predetermined retrieval range of tag acquisition history information and retrieves matched search-tagged observation information (step S87 in the example shown in FIG. 27, step S87' in the example shown in FIG. 28, step S87" in the example shown in FIG. 29, and step S87"' in the example shown in FIG. 31).

The other processing procedures are roughly the same as those in the cell observation information processing system 10 according to the first embodiment.

According to the cell observation information processing system 10 of the second embodiment, the storing section 12 stores, as the tag acquisition history information, tags containing cell identifying information including a cell name, first culture tool identifying information, and second culture tool identifying information acquired by the tag acquiring section 11, as the search tag, in a file separate from the file for storing search-tagged observation information. Therefore, in the search tag acquisition process by the tag acquiring section 11 and the search-tagged observation information retrieval process by the retrieving section 15, a target to be searched for and/or retrieved can be a file that stores tag acquisition history information including no large-byte data, such as a cell image, whereby the efficiency of the search and/or retrieval process can be improved.

Further, according to the cell observation information processing system 10 of the second embodiment, the retrieving section 15 repeats, on the basis of a predetermined acquisition history information retrieval range, the process of searching the second archive section 13-2 using the search criterion acquiredby the search criterion acquiring section 14, retrieving matched tag acquisition history information, then further newly creating a search criterion in which the first culture tool identifying information in the search criterion is replaced with the second culture tool identifying information included in the retrieved tag acquisition history information, re-searching through the second archive section 13-2 using the created search criterion, and retrieving matched tag acquisition history information, and the retrieving section 15 then sequentially searches through the first archive section 13-1 using all the search criteria used to retrieve the tag acquisition history information on the basis of the predetermined retrieval range of tag acquisition history information and retrieves matched search-tagged observation information, whereby not only can the retrieving section 15 retrieve search-tagged observation information on cells having undergone passage of culture from a single cell line over a plurality of generations be embodied, but also the efficiency of the search and/or retrieval process in the processing course of the search-tagged observation information retrieval process by the retrieving section 15 is improved.

Other functions and effects are roughly the same as those in the cell observation information processing system 10 according to the first embodiment.

### Third embodiment

FIGs. 31 are explanatory diagrams that show examples of the data structure of first culture tool identifying information and second culture tool identifying information stored in an archive section in a cell observation information processing system according to a third embodiment of the present invention; FIG. 31(a) shows an example of the data structure of the first culture tool identifying information and the second culture tool identifying information in search-tagged observation information, and FIG. 31(b) shows an example of the data structure of the first culture tool identifying information and the second culture tool identifying information in tag acquisition history information.

In the cell observation information processing system 10 according to the third embodiment, the first culture tool identifying information involves the second culture tool identifying information, as shown, for example, in FIG. 31(a).

In the first culture tool identifying information in each set of data stored in a search-tagged observation information file shown in FIG. 31(a), the numeral at the rightmost digit represents identification information specific to the first culture tool (number specific to culture container, for example), and the numeral at the remaining digits represents the second culture tool identifying information.

Inmore detail, for example, in the first culture tool identifying information "1-1-2" in the data labeled with reference character R7 stored in the search-tagged observation information file shown in FIG. 31 (a), the numeral "2" at the rightmost digit labeled with reference character α1 represents identification information specific to the first culture tool, and the numeral "11" at the two digits labeled with reference character α2 represents the second culture tool identifying information.

The numeral "11" at the two digits labeled with reference character α2 coincides with the value representing the first culture tool identifying information in the data labeled with reference characters R4 and R2 and having a passage number one generation earlier. Further, in the numeral "11" at the two digits labeled with reference character α2, the numeral "1" at the right digit coincides with the value representing identification information specific to the first culture tool in the data labeled with reference characters R4 and R2 and having a passage number one generation earlier, and the numeral "1" at the left digit coincides with the value representing the first culture tool identifying information in the data labeled with reference character R1 and having a passage number two generations earlier.

Further, in the cell observation information processing system 10 according to the third embodiment, the first culture tool identifying information differs from the first culture tool identifying information or the second culture tool identifying information in the cell observation information processing systems 10 according to the first and second embodiments in that no passage number information is included in the data.

The configuration in which the first culture tool identifying information and the second culture tool identifying information are integrated with each other is also applicable to the configuration in which search-tagged observation information is stored in the first archive section 13-1 and tag acquisition history information is stored in the second archive section 13-2, as in the cell observation information processing system 10 according to the second embodiment described above. In this case, the first culture tool identifying information in the tag acquisition history information is configured as shown, for example, in FIG. 31(b).

The retrieving section 15 is configured to search through the archive section 13 using a search criterion acquired by the search criterion acquiring section 14, retrieve matched search-tagged observation information, then further search through the archive section 13 sequentially using search criteria in which the second culture tool identifying information of respective generations involved in the first culture tool identifying information in the search criterion replaces and serves as first culture tool identifying information in search criterion, and retrieve matched search-tagged observation information.

The other configurations are roughly the same as those of the cell observation information processing system 10 according to the first embodiment.

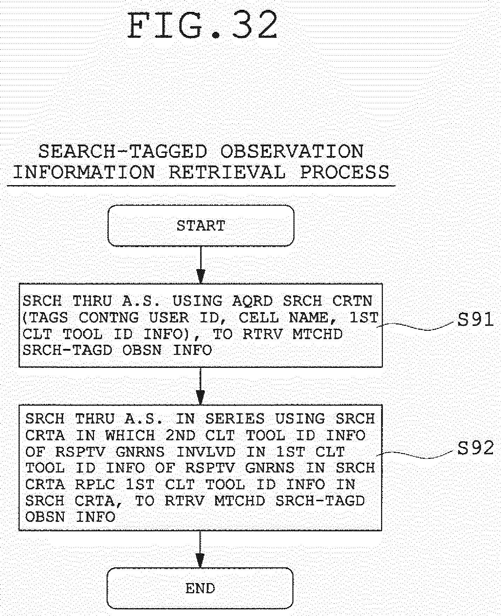

In the thus configured cell observation information processing system 10 according to the third embodiment, the processing procedure of the search-tagged observation information retrieving process (step S12 in FIG. 16) at the stage of searching for and/or retrieving observation information on desired cells from the archive section and checking the condition of the cells differs from the processing procedure in the cell observation information processing system 10 according to the first embodiment. The different processing procedure will be described with reference to FIG. 32.

FIG. 32 is a flowchart that shows an example of the processing procedure of the search-tagged observation information retrieval process on cells having undergone passage of culture from a single cell line over a plurality of generations, at the stage of searching for and/or retrieving observation information on desired cells from the archive section using the cell observation in format ion processing system in FIGs. 31.

In the process of acquiring search-tagged observation information, the retrieving section 15 searches through the archive section 13 using the search criterion (tags containing user ID, cell name, and first culture tool identifying information) acquired by the search criterion acquiring section 14 and retrieves matched search-tagged observation information (step S91). The retrieving section 15 further searches through the archive section 13 sequentially using search criteria in which the second culture tool identifying information of respective generations involved in the first culture tool identifying information in the search criterion replaces and serves as first culture tool identifying information in search criterion and retrieves matched search-tagged observation information (step S92).

The other processing procedures are roughly the same as those in the cell observation information processing system 10 according to the first embodiment.

According to the cell observation information processing system 10 of the third embodiment, since the first culture tool identifying information and the second culture tool identifying information are integrated with each other, data involved in the first culture tool identifying information on cells of a generation having a certain passage number allows identification of first culture tool identifying information and second culture tool identifying information on cells of all generations that link with one another retrospectively from the generation of the passage number concerned.

The retrieving section 15 therefore does not need, whenever carrying out the process of searching through the archive section 13 using a search criterion and retrieving matched search-tagged observation information, to carry out the process of acquiring the second culture tool identifying information included in the retrieved search-tagged observation information but can continuously search through the archive section 13 using search criteria in which second culture tool identifying information in respective generations identified in the first culture tool identifying information data contained in the search criterion acquired by the search criterion acquiring section 14 replaces and serves as first culture tool identifying information in the search criteria and retrieve matched search-tagged observation information. Further, the search criterion used by the retrieving section 15 to retrieve, back to earlier generations, search-tagged observation information from the archive section 13 requires no information on the passage number. As a result, the efficiency of the process of retrieving observation information on cells having undergone passage of culture from a single cell line over a plurality of generations is significantly improved.

Other functions and effects are roughly the same as those in the cell observation information processing system 10 according to the first embodiment.

The cell observation information processing system according to each of the embodiments of the present invention is further preferably configured so that the presenting section 18 displays a tree diagram on a cell culture container basis, for example, on the basis of a group of pieces of observation information having the same user ID, cell name, passage number, first culture tool identifying information, and second culture tool identifying information by using a database in the archive section where observation information or tag acquisition history information is stored.

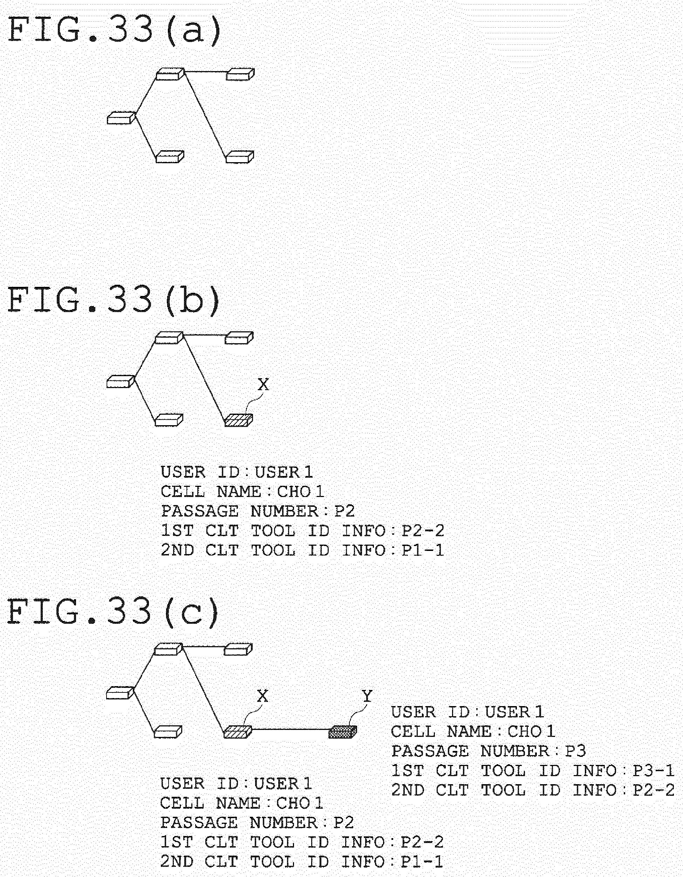

FIGs. 33 are explanatory diagrams that schematically show an example of a cell culture container tree diagram on container basis by using tagged observation information stored in the archive section and on the basis of a group of pieces of observation information having the same user ID, cell name, passage number, first culture tool identifying information, and second culture tool identifying information.

In the cell observation information processing system according to each of the embodiments of the present invention to which the configuration with the presenting section 18 displaying the tree diagram on container basis shown in FIGs. 33 is applied, for example, in performing the process of observing cells for the first time after passage work to acquire observation information and adding a new container (culture tool), for example, in a case where a tree diagram, such as that shown in FIG. 33(a), is displayed, when the user presses a new container addition button, which is not shown, the tag acquiring section 11 searches for search-tagged observation information using the same user ID and the cell name. The tag acquiring section 11 then retrieves search-tagged observation information having passage presence/absence information indicating passage work and having the latest observation information acquisition time point in the date/time information among pieces of matched search-tagged observation information. The tag acquiring section 11 then causes the presenting section 18 to display, as a possible passage source, a container having the same user ID, cell name, passage number, first culture tool identifying information and second culture tool identifying information as those recorded in the retrieved search-tagged observation information, for example, the container X shown in FIG. 33(b) in the highlighted mode.

At this point, when the user uses selection means, which is not shown, to select the container X shown in FIG. 33(b) as the passage-source cell container, the tag acquiring section 11 causes the presenting section 18 to additionally display a new passage-destination container Y, which links with the passage-source container X, as shown in FIG. 33(c). At the same time, the tag acquiring section 11 acquires pieces of information (tags including user ID, cell name, passage number, first culture tool identifying information, and second culture tool identifying information) related to the additionally displayed container Y together and causes the presenting section 18 to display the information.

Further, to assign a search tag to observation information and store the resultant observation information, when the user selects the additionally displayed new container Y by using the selecting means, which is not shown, on the tree diagram shown in FIG. 33(c), the storing section 12 assigns the tags related to the container together to the observation information.

When the presenting section 18 is configured to display a tree diagram on a cell culture container basis, the second culture tool identifying information can be explicitly set, whereby the number of errors in the setting made by the user can be reduced.

Further, since tags explicitly related to a container can be assigned together to observation information, the number of errors in user's tag setting can be reduced, as compared with a configuration in which a search tag to be assigned to observation information is individually set.

The embodiments of the cell observation information processing system according to the present invention have been described. Each of the cell observation information processing systems may instead be formed, for example, of a cell observation information processing program for causing a computer provided in the installation 1 to function as tag acquiring means for acquiring, as a search tag for searching for observation information in response to an acquisition order at one time point to include a cell image and activity data indicating cell activity, tags containing cell identifying information including a cell name, first culture tool identifying information for distinguishing a first culture tool that is a culture tool for culturing cells under observation from another culture tool for culturing cells having the same passage number as that of the cells cultured in the first culture tool, and second culture tool identifying information for identifying a second culture tool that is a culture tool for culturing cells that serve as a passage source of the cells cultured in the first culture tool, storing means for assigning the search tag acquired by the tag acquiring means to observation information acquired in response to an acquisition order at each time point and storing search-tagged observation information to which the search tag is assigned in an archive section, search criterion acquiring means for acquiring, as the search criterion, tags including the cell name of first cells and the first culture tool identifying information, and retrieving means for searching through the archive section using the search criterion acquired by the search criterion acquiring means and retrieving search-tagged observation information on cells having undergone passage of culture from a single cell line over a plurality of generations.

Further, the cell observation information processing system according to each of the embodiments of the present invention does not necessarily have the configuration in which the cell observation information processing program is installed in the computer provided in the installation 1 and may instead have a configuration in which the cell observation information processing program is recorded on a recording medium readable by the computer provided in the installation 1.

Further, the cell observation information processing system according to each of the embodiments of the present invention does not necessarily have the configuration in which the tag acquiring section 11, the storing section 12, the search criterion acquiring section 14, and the retrieving section 15 are provided in the single installation 1, as shown in FIG. 1, and may instead have a configuration in which the tag acquiring section, the storing section, the search criterion acquiring section, and the retrieving section are separately provided in a plurality of apparatuses. Still instead, a cell observation information processing system according to another embodiment of the present invention may have a configuration in which the archive section 13 is provided together with the tag acquiring section 11, the storing section 12, the search criterion acquiring section 14, and the retrieving section 15.

Further, the embodiments described above have the configuration in which tags are manually acquired and assigned by the user, that is, the configuration in which the presenting section, such as a screen, accepts user's manual input and the tag acquiring section acquires the tags accepted by the presenting section and assigns the acquired tag to observation information, such as an image, but the configuration for acquiring and assigning tags may instead be any of the configurations in the following variations:

### Variation 1: Configuration in which apparatus automatically acquires and assigns tag

The tag acquiring section may store a tag having a content designated in advance in the apparatus and acquire and assign the tag stored in the apparatus when the tag is assigned. For example, in a case where the user exclusively operates a certain apparatus, the user ID of the user is stored in the apparatus in advance.

According to Variation 1, the apparatus automatically acquires and assigns a tag, whereby user's burden of inputting a tag is reduced.

### Variation 2: Configuration in which tag is acquired and assigned in user's other input aspects

The tag acquiring section may be configured to acquire and assign a tag in response, for example, to user's voice input, user's eye gaze input or user's gesture input.

For example, in the case of voice input, the tag acquiring section may use the voice analysis technology disclosed, for example, in Japanese Patent Kokai No. 2012-252181 to convert voice information as a tag input by the user in the form of voice into letter string information as a tag and assign the letter string information as a tag to observation information, such as an image.

Further, for example, in the case of eye gaze input, relation information that relates the direction in which user's eye gaze moves to letters corresponding to the direction may be stored in the apparatus, and the tag acquiring section may detect the direction of user's eye gaze with an eye gaze detector, such as an eye gaze detecting sensor, and acquire the letters related to the direction as a tag on the basis of the detected direction of user's eye gaze and the relation information.

Further, for example, in the case of gesture input, relation information that relates the direction in which any of user's limbs, trunk, and other body parts moves to letters corresponding to the direction maybe stored in the apparatus, and the tag acquiring section may detect the direction in which user's body part moves with a gesture detector, such as a gesture detecting sensor, and acquire the letters corresponding to the direction as a tag on the basis of the detected direction of user's body part and the relation information.

In the present variation, examples of user's s other input aspects, such as user's voice input and user's eye gaze input, are presented, but user's input aspect is not limited thereto. For example, a tag may be acquired and assigned in response, for example, to brain wave input.

The embodiments of the present invention and the variations thereof have been described above, but the present invention is not limited to the configurations described in the embodiments and the variations thereof. At the stage of carrying out the present invention, each component in the present invention can be embodied in a modified form to the extent that the modification does not change the substance of the present invention. Further, a plurality of components described in the embodiments and the variations thereof can be combined with each other as appropriate to derive a variety of aspects of the present invention. For example, the components described in the embodiments and the variations thereof may be partially omitted, or components described in different embodiments and variations thereof may be combined with each other as appropriate. A variety of changes and applications are thus conceivable to the extent that the substance of the present invention is not changed.

### INDUSTRIAL APPLICABILITY

The cell observation information processing system, the cell observation information processing method, and the cell observation information processing program according to the present invention are useful not only in a field where the condition of cells used in research on a living body is required to be controlled by using observation information on the cells acquired by an observation information acquiring apparatus, such as a microscope, but also in a field where the condition of a living body that changes with time is required to be controlled.

### LIST OF REFERENCE SYMBOLS

- 1: Cell observation information installation
- 10: Cell observation information processing system
- 11: Tag acquiring section
- 12: Storing section
- 13: Archive section
- 13-1: First archive section
- 13-2: Second archive section
- 14: Search criterion acquiring section
- 15: Retrieving section
- 16: Tag selecting section
- 18: Presenting section
- 19: Outputting section
- 20: Observation information acquiring section
- 21: Tag inputting section

## Claims

1. A cell observation information processing system including:
a tag acquiring section that acquires, as a search tag for searching for observation information that has been acquired in response to an acquisition order at one time point via an observation information acquiring section to include a cell image and activity data indicating cell activity, tags containing cell identifying information including a cell name, first culture tool identifying information for distinguishing a first culture tool that is a culture tool for culturing cells under observation from another culture tool for culturing cells having a same passage number as a passage number of the cells cultured in the first culture tool, and second culture tool identifying information for identifying a second culture tool that is a culture tool for culturing cells that serve as a passage source of the cells cultured in the first culture tool;
a storing section that assigns the search tag acquired by the tag acquiring section to each piece of the observation information acquired in response to the acquisition order at each time point and stores search-tagged observation information to which the search tag is assigned in an archive section;
a search criterion acquiring section that acquires, as a search criterion, tags containing a cell name and a piece of first culture tool identifying information; and
a retrieving section that searches through the archive section using the search criterion acquiredby the search criterion acquiring section and retrieves search-tagged observation information on cells having undergone passage of culture from a single cell line over a plurality of generations.

2. The cell observation information processing system according to claim 1,
wherein the retrieving section
searches through the archive section using the search criterion acquired by the search criterion acquiring section and retrieves matched search-tagged observation information, and then
repeats, based on a predetermined retrieval range of observation information, a process of re-searching through the archive section using a search criterion in which the second culture tool identifying information included in the retrieved search-tagged observation information replaces and serves as the first culture tool identifying information in the search criterion.

3. The cell observation information processing system according to claim 2,
wherein the predetermined retrieval range of observation information is configured so that the observation information is retrieved until search-tagged observation information having no second culture tool identifying information recorded therein is retrieved.

4. The cell observation information processing system according to claim 2,
wherein
the search tag further contains date/time information on observation information acquisition time point, and
the predetermined retrieval range of observation information is configured to be defined by a range, designated by user's s operation, of date/time information on observation information acquisition time point.

5. The cell observation information processing system according to claim 2,
wherein
the search tag further contains a passage number of the cells cultured in the first culture tool, and
the predetermined retrieval range of observation information is configured to be defined by a range of passage number designated by user's operation.

6. The cell observation information processing system according to claim 1,
wherein the first culture tool identifying information is formed of a passage number of the cells cultured in the first culture tool and identification information specific to the first culture tool.

7. The cell observation information processing system according to claim 1,
wherein the second culture tool identifying information is formed of a passage number of the cells cultured in the second culture tool and identification information specific to the second culture tool.

8. The cell observation information processing system according to claim 1,
wherein
the search tag further includes a passage number of the cells cultured in the first culture tool, and
the tag acquiring section
searches through the archive section using the cell name designated by user's operation, retrieves, from matched search-tagged observation information, search-tagged observation information having a largest passage number of the cells cultured in the first culture tool, and outputs to a presenting section, as pieces of possible first culture tool identifying information to be acquired as a part of the search tag, pieces of first culture tool identifying information included in the retrieved search-tagged observation information, in a form of a list,
acquires, as a part of the search tag, a piece of first culture tool identifying information selected by user's operation among the pieces of possible first culture tool identifying information output to the presenting section in the form of a list, and
automatically acquires, as a part of the search tag, the second culture tool identifying information included in the search-tagged observation information having the piece of first culture tool identifying information selected by user's operation.

9. The cell observation information processing system according to claim 1,
wherein
the tag acquiring section
acquires, as a part of the search tag, the first culture tool identifying information designated by user's operation, and
searches through the archive section using the cell name and the first culture tool identifying information designated by user's operation and, if matched search-tagged observation information is present, automatically acquires, as a part of the search tag, second culture tool identifying information included in the search-tagged observation information.

10. The cell observation information processing system according to claim 1,
wherein
the first culture tool identifying information is configured to involve the second culture tool identifying information, and
after searching through the archive section using the search criterion acquired by the search criterion acquiring section and retrieving matched search-tagged observation information, the retrieving section further searches through the archive section sequentially using search criteria in which the second culture tool identifying information of respective generations involved in the first culture tool identifying information in the search criterion replaces and serves as first culture tool identifying information in search criterion, to retrieve matched search-tagged observation information.

11. The cell observation information processing system according to claim 1,
wherein the storing section stores, as tag acquisition history information, tags containing the cell identifying information including the cell name, the first culture tool identifying information, and the second culture tool identifying information acquired as the search tag by the tag acquiring section, in a file separate from a file for storing the search-tagged observation information in the archive section.

12. The cell observation information processing system according to claim 11,
wherein
after searching through the archive section using the search criterion acquired by the search criterion acquiring section and retrieving matched tag acquisition history information,
the retrieving section further repeats, based on a predetermined retrieval range of tag acquisition history information, a process of newly creating a search criterion in which the second culture tool identifying information included in the retrieved tag acquisition history information replaces and serves as the first culture tool identifying information in the search criterion, re-searching through the archive section using the created search criterion, and retrieving matched tag acquisition history information, and
then sequentially searches through the archive section using all the search criteria that were used to retrieve the tag acquisition history information based on the predetermined retrieval range of tag acquisition history information and retrieves matched search-tagged observation information.

13. The cell observation information processing system according to claim 12,
wherein the predetermined retrieval range of tag acquisition history information is configured so that the tag acquisition history information is retrieved until tag acquisition history information having no second culture tool identifying information recorded therein is retrieved.

14. The cell observation information processing system according to claim 12,
wherein
the search tag and the tag acquisition history information each further include date/time information on observation information acquisition time point, and
the predetermined retrieval range of tag acquisition history information is configured to be defined by a range, determined by user's operation, of date/time information on observation information acquisition time point.

15. The cell observation information processing system according to claim 12,
wherein
the search tag and the tag acquisition history information each further include a passage number of the cells cultured in the first culture tool, and
the predetermined retrieval range of tag acquisition history information is configured to be defined by a range of passage number designated by user's operation.

16. The cell observation information processing system according to claim 11,
wherein
the search tag further contains a passage number of the cells cultured in the first culture tool, and
the tag acquiring section
searches through the archive section using the cell name designated by user's operation, retrieves, from matched tag acquisition history information, tag acquisition history information having a largest passage number of the cells cultured in the first culture tool, and outputs to a presenting section, as pieces of possible first culture tool identifying information to be acquired as a part of the search tag, pieces of first culture tool identifying information included in the retrieved tag acquisition history information, in a form of a list,
acquires, as a part of the search tag, a piece of first culture tool identifying information selected by user's operation among the pieces of possible first culture tool identifying information output to the presenting section in the form of a list, and
automatically acquires, as a part of the search tag, the second culture tool identifying information included in the tag acquisition history information having the first culture tool identifying information selected by user's operation.

17. The cell observation information processing system according to claim 11,
wherein
the tag acquiring section
acquires, as a part of the search tag, the first culture tool identifying information designated by user's operation, and
searches through the archive section using the cell name and the first culture tool identifying information designated by user's operation and, if matched tag acquisition history information is present, automatically acquires, as a part of the search tag, the second culture tool identifying information included in the tag acquisition history information.

18. A cell observation information processing method comprising:
a tag acquiring step of acquiring, as a search tag for searching for observation information that has been acquired in response to an acquisition order at one time point to include a cell image and activity data indicating cell activity, tags containing cell identifying information including a cell name, first culture tool identifying information for distinguishing a first culture tool that is a culture tool for culturing cells under observation from another culture tool for culturing cells having a same passage number as a passage number of the cells cultured in the first culture tool, and second culture tool identifying information for identifying a second culture tool that is a culture tool for culturing cells that serve as a passage source of the cells cultured in the first culture tool;
a storage step of assigning the search tag acquired in the tag acquiring step to the observation information acquired in response to the acquisition order at each time point and storing search-tagged observation information to which the search tag is assigned in an archive section;
a search criterion acquiring step of acquiring, as a search criterion, tags containing a cell name and a piece of first culture tool identifying information; and
a retrieval step of searching through the archive section using the search criterion acquired in the search criterion acquiring step and retrieving search-tagged observation information on cells having undergone passage of culture from a single cell line over a plurality of generations.

19. A cell observation information processing program that causes a computer to function as:
tag acquiring means for acquiring, as a search tag for searching for observation information that has been acquired in response to an acquisition order at one time point to include a cell image and activity data indicating cell activity, tags containing cell identifying information including a cell name, first culture tool identifying information for distinguishing a first culture tool that is a culture tool for culturing cells under observation from another culture tool for culturing cells having a same passage number as a passage number of the cells cultured in the first culture tool, and second culture tool identifying information for identifying a second culture tool that is a culture tool for culturing cells that serve as a passage source of the cells cultured in the first culture tool;
storing means for assigning the search tag acquired by the tag acquiring means to the observation information acquired in response to an acquisition order at each time point and storing search-tagged observation information to which the search tag is assigned in an archive section;
search criterion acquiring means for acquiring, as a search criterion, tags containing a cell name of the first cells and a piece of first culture tool identifying information; and
retrievingmeans for searching through the archive section using the search criterion acquiredby the search criterion acquiring means and retrieving search-tagged observation information on cells having undergone passage of culture from a single cell line over a plurality of generations.
